(19) Europäisches Patentamt
European Patent Office
Office européen des brevets

(11) Publication number: **0 053 387**
**A1**

# (12) EUROPEAN PATENT APPLICATION

(21) Application number: **81110039.5**

(22) Date of filing: **01.12.81**

(51) Int. Cl.³: **C 07 D 205/08**, C 07 F 7/10, C 07 D 417/12

(30) Priority: **03.12.80 PC /JP80/002 93**

(43) Date of publication of application: **09.06.82 Bulletin 82/23**

(84) Designated Contracting States: **AT BE CH DE FR GB IT LI LU NL SE**

(71) Applicant: **Takeda Chemical Industries, Ltd., 27, Doshomachi 2-Chome, Higashi-ku Osaka, 541 (JP)**

(72) Inventor: **Matsuo, Taisuke, 2-Minamiai 2-chome, Ibaraki-shi Osaka 567 (JP)**
Inventor: **Masuya, Hirotomo, 1-19, Matsuodai 4-chome Inagawa-cho, Kawabe-gun Hyogo 666-02 (JP)**
Inventor: **Noguchi, Noriyoshi, 6-6, Taisho 1-chome, Kashihara-shi Osaka 582 (JP)**
Inventor: **Ochiai, Michihiko, 23-13, Senriyama Higashi 1-chome, Suita-shi Osaka 565 (JP)**

(74) Representative: **von Kreisler, Alek, Dipl.-Chem. et al, Deichmannhaus am Hauptbahnhof, D-5000 Köln 1 (DE)**

**(54) Method of preparing 1-sulfo-2-oxoazetidine derivatives and intermediates therefor.**

(57) 1-Sulfo-2-oxoazetidine derivatives represented by the formula (I)

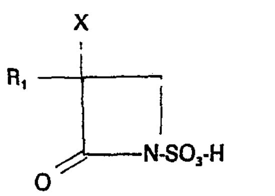

(I)

wherein $R_1$ stands for an amino group which may optionally be acylated or protected, and X stands for hydrogen or methoxy, is produced by subjected a 1-t-butyl or isopropylsilyl derivative of a compound represented by the formula (II)

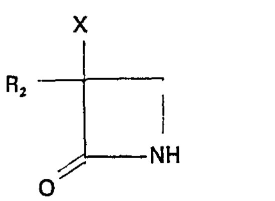

(II)

wherein $R_2$ stands for an amino group which is acylated or protected, and X is of the same meaning as defined above, to sulfonation, followed by, when $R_2$ is a protected amino group, removing the protective group, if necessary.

The compounds represented by the formula (I) have an excellent antibacterial and β-lactamase-inhibitory activity, thus being useful as drugs for humans and domestic animals.

EP 0 053 387 A1

# METHOD OF PREPARING 1-SULFO-2-OXOAZETIDINE DERIVATIVES AND INTERMEDIATES THEREFOR

This invention relates to a method of preparing 1-sulfo-2-oxoazetidine derivatives having antimicrobial or β-lactamase inhibitory activity.

Recently, there have been reported various types of 2-oxoazetidine compounds as having antimicrobial or β-lactamase inhibitory activity. For example, in a British Patent No. 1,519,495, there are disclosed 2-acylamino-azetidine derivatives prepared by subjecting a 3-amino-1-substituted-2-oxoazetidine compound to acylation at 3-position, but those derivatives are ones whose 1-position is substituted by cyano or carboxy-substituted aliphatic hydrocarbon residue.

In the search for novel and useful azetidine derivatives, the present inventors found that azetidine derivatives having a sulfo group at the 1-position satisfy its demand, and also that such azetidine derivatives can be easily produced by subjecting an azetidine derivtive, which has been silylated at 1-position, to sulfonation.

These findings were followed by further studies which have culminated in the completion of the present invention.

The present invention is directed to:

(1) a method of preparing 1-sulfo-2-oxoazetidine derivatives represented by the formula:

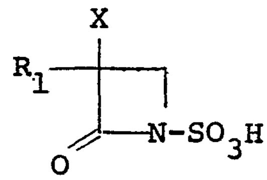

(I)

wherein $R_1$ stands for an amino group which may optionally be acylated or protected, and X stands for hydrogen or methoxy, which comprises subjecting a 1-t-butyl or isopropyl silyl derivative of a compound represented by the formula:

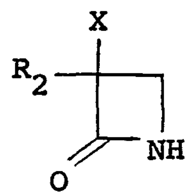

(II)

wherein $R_2$ stands for an amino group which is acylated or protected, and X is of the same meaning as defined above, to sulfonation, followed by, when $R_2$ is a protected amino group, removing the protective group, if necessary.

(2) a method of preparing 1-sulfo-2-oxoazetidine derivatives represented by the formula:

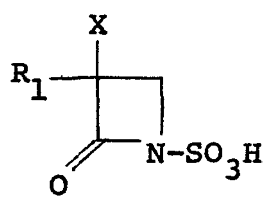

(I)

wherein $R_1$ stands for an amino group which may optionally be acylated or protected, and X stands for hydrogen or methoxy, which comprises reacting a compound of the formula:

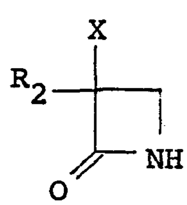

(II)

wherein $R_2$ stands for an amino group which is acylated or protected, and X is of the same meaning as defined above, with a t-butyl or isopropylsilyl compound to give a 1-t-butyl or isopropylsilyl derivative of the compound (II),

and then, when $R_2$ is an acylated amino group, subjecting the 1-t-butyl on isopropylsilyl derivatives of the compound (II) to sulfonation,

when $R_1$ is a protected amino group, removing the protective group, acylating the amino group and subjecting the 1-t-butyl or isopropylsilyl derivative of the compound (II) to sulfonation,

or when $R_1$ is a protective amino group, subjecting the 1-t-butyl or isopropylsilyl derivative of the compound (II) to sulfonation without removing the protective group, followed by removing the protective group, if necessary.

(3) 1-t-butyl or isopropylsilyl derivatives of a compound of the formula:

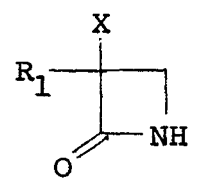

(II)

wherein $R_1$ stands for an amino group which may optionally be acylated or protected, and X stands for hydrogen or methoxy.

(4) a method of preparing a 1-t-butyl or isopropylsilyl derivatives of a compound of the formula:

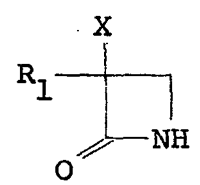

(II)

wherein $R_1$ stands for an amino group which may optionally be acylated or protected, and X stands for hydrogen or methoxy,

which comprises reacting a compound of the formula:

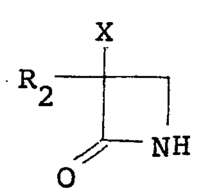

(II)

wherein $R_2$ stands for an amino group which is acylated or protected, and X is of the same meaning as defined above, with a t-butyl or isopropylsilyl compound, followed by removing the protective group of $R_2$, if necessary, when $R_2$ is a protected amino group.

In the above general formulas, the acyl moiety of the acylated amino group, $R_1$ or $R_2$, may be one of the acyl groups on 6-amino of known penicillin derivatives or on 7-amino of known cephalosporin derivatives.

As examples of such acyl groups, there may be mentioned (1) a group of the formula:

$$R_6\text{-CO-}$$

wherein $R_6$ is a lower alkyl group, phenyl which may optionally be substituted, benzoyl which may optionally be substituted, or a heterocyclic group which may optionally be substituted,

(2) a group of the formula:

$$R_7\text{-NH-}\underset{\displaystyle R_9}{\text{CH}}\text{-CO-}$$

wherein $R_7$ is hydrogen, an amino acid residue which may optionally be substituted, an amino-protecting group, a group of $R_8\text{-}(CH_2)_n\text{-CO-}$ [where $R_8$ is a heterocyclic group which may optionally be substituted, phenyl which may optionally be substituted, a lower alkyl group which may optionally be substituted, phenylthio which may optionally be substituted or a lower alkylthio group; n is an integer of 0 to 4; and the $-(CH_2)_n-$ group may be substituted], a group of $\genfrac{}{}{0pt}{}{R_8'}{R_8''}\!>\!N\text{-CO-}$ [where $R_8'$ and $R_8''$ may be the same or different and each is hydrogen, a lower alkyl group, a lower alkylcarbamoyl group, phenylcarbonyl which may optionally be substituted, or sulfo] or a group of $R_8'''\text{-}SO_2\text{-}$ [where $R_8'''$ is a lower alkyl group which may optionally be substituted]; $R_9$ is hydrogen, a lower alkyl group which may optionally be substituted, a phenyl group which may optionally be substituted, a heterocyclic group which may optionally be substituted, a cycloalkenylene group, a

heterocycle-carbonylamino group which may optionally be substituted or be interrupted by an alkylene group,

(3) a group of the formula:

$$R_{10}-R_{11}-CO-$$

wherein $R_{10}$ is a group of the formula: $R_{12}-\underset{\underset{N-X'-R_{13}}{\|}}{C}-$ {where X' is oxygen or sulfur, $R_{12}$ is a heterocyclic group which may optionally be substituted or phenyl which may optionally be substituted, $R_{13}$ is hydrogen, phenyl which may optionally be substituted, a lower acyl group which may optionally be substituted, a lower alkyl group, or a group of the formula $-R_{14}-R_{15}$ (where $R_{14}$ is a lower alkylene or a lower alkenylene group, $R_{15}$ is carboxyl, an ester thereof or a heterocyclic group)}, $R_{11}$ is a single bond or a group of the formula $-CO-NH-\underset{\underset{R_{16}}{|}}{CH}-$ (where $R_{16}$ is a lower alkyl group, phenyl which may optionally be substituted or a heterocyclic group which may optionally be substituted),

(4) a group of the formula:

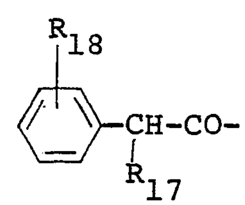

wherein $R_{17}$ is hydroxyl, hydroxysulfonyloxy, carboxyl, sulfamoyl which may optionally be substituted, sulfo, phenoxycarbonyl which may optionally be substituted, benzyloxycarbonyl, formyloxy, or phthalimide group; $R_{18}$ is hydrogen, a lower alkyl group, a lower alkoxy group, halogen, azide, nitro or hydroxyl, or

(5) a group of the formula:

$$R_{19}-R_{20}-CH_2-CO-$$

wherein $R_{19}$ is cyano, a phenyl group which may optionally be substituted, phenoxy which may optionally be substituted, a lower alkyl group which may optionally be substitued, an

alkenylene group which may optionally be substituted, or a heterocyclic group which may optionally be substituted, $R_{20}$ is a single bond or -S-.

The lower alkyl group $R_6$ preferably contains 1 to 6 carbon atoms.

The substituents on said optionally substituted phenyl group represented by $R_6$ may for example be lower alkyl of 1 to 3 carbon atoms, lower alkoxy of 1 to 3 carbon atoms, halogen, nitro, amino or the like.

The substituents on said optionally substituted benzoyl represented by $R_6$ may for example be lower alkyl of 1 to 3 carbon atoms, lower alkoxy of 1 to 3 carbon atoms, halogen, amino or the like.

The heterocyclic moiety of the optionally substituted heterocyclic group $R_6$ is a 5- or 6-membered heterocyclic group including 1 to 2 nitrogen atoms, which may optionally include a single oxygen atom. Examples of such heterocyclic group include isoxazolyl, piperazinyl, imidazolinyl, etc. The substituents on such heterocyclic groups may for example be lower alkyl of 1 to 3 carbon atoms, lower alkoxy of 1 to 3 carbon atoms, halogen, nitro, amino, oxo, thioxo, or phenyl which may optionally be substituted.

The amino acid residue for the optionally substituted amino acid residue $R_7$ may for example be glycyl, alanyl, valyl, leucyl, isoleucyl, seryl, threonyl, cysteinyl, cystyl, methionyl, α- or β-aspartyl, α- or γ-glutamyl, lysyl, arginyl, phenylalanyl, phenylglycyl, tyrosyl, histidyl, tryptophyl, prolyl, etc. The substituents that may be present on such amino acid residues may for example be amino, lower alkylamino, an amino-protecting group, carbamoyl, methylcarbamoyl, sulfamoyl, benzyl, 4-ethyl-2,3-dioxo-1-piperazinecarbonyl, 4-ethyl-2,3-dioxo-1-piperazine-carbonylamino, etc. The lower alkyl moiety of said lower alkylamino preferably contains 1 to 3 carbon atoms. The protective group on this amino group may be one of those amino-protecting groups mentioned hereinafter.

The amino-protecting group $R_7$ may be one of those amino-protecting groups mentioned hereinafter.

The heterocyclic moiety of the optionally substitued heterocyclic group $R_8$ in the group of $R_8-(CH_2)_n-CO-$ may for example be a 5- or 6-membered heterocyclic group including one sulfur, nitrogen or oxygen atom, a 5- to 6-membered heterocyclic group including 2 to 4 nitrogen atom, or a 5- to 6-membered heterocyclic group including 1 to 2 nitrogen and one sulfur or oxygen atom, and these heterocyclic groups may each be fused to a 6-membered cyclic group including not more than 2 nitrogen atoms, a benzene ring or a 5-membered cyclic group including one sulfur atom.

Examples of the heterocyclic group $R_8$ include 2-pyridyl, 3-pyridyl, 4-pyridyl, pyrimidinyl, pyrazinyl, pyridazinyl, piperazinyl, pyrazolinyl, imidazolidinyl, thiazolyl, isothiazolyl, oxazolyl, isoxazolyl, pyrido[2,3-d]pyrimidinyl, benzopyranyl, 1,8-naphthylidinyl, 1,5-naphthylidinyl, 1,6-naphthylidinyl, 1,7-naphthylidinyl, 2,7-naphthylidinyl, 2,6-naphthylidinyl, quinolyl, thieno[2,3-b]pyridinyl, tetrazolyl, thiadiazolyl, oxadiazolyl, triazinyl, thienyl, pyrrolyl, furyl, etc.

The substituents on the optionally substituted heterocyclic group $R_8$ may for example be substituted or unsubstituted alkyl group of 1 to 12 carbon atoms, lower alkoxy group of 1 to 3 carbon atoms, hydroxyl, oxo, thioxo, aldehyde, trifluoromethyl, amino, halogen, lower $(C_{1-3})$ alkylsulfonyl, 2,6-dichlorophenyl, coumarin-3-carbonyl, 4-formyl-1-piperazinyl, pyrrolaldoimino, furanaldoimino, thiophenaldoimino, mesyl, an amino-protecting group, alkylcarbonyl $(C_{2-4})$ amino which may be substituted by halogen. The amino-protecting group may be those mentioned hereinafter. The substituents optionally present on said $(C_{1-12})$ alkyl group may for example be phenyl, halogen, hydroxyl, dialkylamino, etc. The alkyl moiety of said dialkylamino is preferably lower $(C_{1-3})$ alkyl.

The substituents on the optionally substituted phenyl

group $R_8$ may for example be lower alkyl of 1 to 3 carbon atoms, lower alkoxy of 1 to 3 carbon atoms, halogen, hydroxyl, amino, etc.

The lower alkyl moiety of the lower alkyl group which may optionally be substituted represented by $R_8$ may for example be alkyl having 1 to 3 carbon atoms. The substituents on the optionally substituted lower alkyl may for example be carboxyl, amino, carbamoyl or ureido.

The substituents on optionally substituted phenylthio $R_8$ may for example be lower ($C_{1-3}$) alkyl, lower ($C_{1-3}$) alkoxy halogen, hydroxyl, amino, etc.

The lower alkyl moiety of lower alkylthio $R_8$ preferably contains 1 to 3 carbon atoms.

The substituents which may be present on the group $-(CH_2)_n-$ may for example be amino or a group of $-NH-CO-R_8''''$ [where $R_8''''$ is amino or a substituted or unsubstituted piperazinyl group]. The substituents on the optionally substituted piperazinyl group $R_8''''$ may for example be lower ($C_{1-3}$) alkyl, lower ($C_{1-3}$) alkoxy, hydroxyl, oxo, thioxo, halogen, etc.

The lower alkyls $R_8'$ and/or $R_8''$ preferably contain 1 to 3 carbon atoms. The lower alkyl moiety of said lower alkylcarbamoyl is preferably a group of 1 to 3 carbom atoms. The substituents on the optionally substituted phenylcarbonyl group may for example be lower ($C_{1-3}$) alkyl, lower ($C_{1-3}$) alkoxy, halogen, hydroxyl, hydroxysulfonyloxy, benzyloxy, etc.

The lower alkyl moiety of said optionally substituted lower alkyl group $R_8'''$ in $R_8'''-SO_2-$ preferably contains 1 to 6 carbon atoms and the substituents may be present in one or two positions and may for example be amino, carboxyl, benzyloxycarbonyl, protected amino, etc. The protective group on said protected amino may be one of those mentioned hereinafter as amino-protecting groups.

The lower alkyl moiety of the optionally substituted lower alkyl $R_9$ preferably contains 1 to 3 carbon atoms, the

substituents being, for example, phenyl, carbamoyl, methylcarbamoyl, methylthio, thienylacetamido, ethoxycarbonylmethylcarbamoyl, N-methyltetrazolylthio, halogen, sulfamoyl, etc.

The substituents on optionally substituted phenyl $R_9$ may for example be lower $(C_{1-3})$ alkyl, lower $(C_{1-3})$ alkoxy, halogen, hydroxyl, hydroxysulfonyloxy, benzyloxy, benzoyloxy, trimethylsilyl, $(C_{2-10})$ alkylcarbonyl, etc.

The heterocyclic ring moiety of optionally substituted heterocyclic group $R_9$ includes among others five-membered heterocyclic groups containing one sulfur, nitrogen or oxygen atom, five-membered heterocyclic groups containing 1-2 nitrogen atoms and one sulfur or oxygen atom, and 5- or 6-membered heterocyclic groups containing 2-4 nitrogen atoms. Examples of such heterocyclic groups are thiazolyl, isothiazolyl, oxazolyl, isoxazolyl, thienyl, furyl, pyrrolyl, imidazolyl, pyrazinyl, pyrimidinyl, pyridazinyl, piperazinyl, triazinyl, tetrazolyl, thiadiazolyl, oxadiazolyl, etc. The substituents on said heterocyclic groups include among others lower $(C_{1-3})$ alkyl, lower $(C_{1-3})$ alkoxy, halogen, hydroxyl, nitro, hydroxysulfonyloxy, amino, and $(C_{2-4})$ alkylcarbonylamino which may be substituted by halogen.

The cycloalkenylene $R_9$ preferably has a 5- or 6-membered ring, and is, for example, cyclohexenyl or cyclohexadienyl.

The heterocyclic moiety of the heterocycle-carbonylamino group $R_9$ which may be substituted and/or interrupted by an alkylene chain may be a 6-membered heterocyclic group containing two nitrogen atoms, such as piperazinyl, which may have such a substituent as $(C_{1-12})$ alkyl, lower $(C_{1-3})$ alkoxy, oxo, thioxo or amino. The alkylene chain preferably contains 1-3 carbon atoms, and is, for example, methylene, ethylene or n-propylene.

The heterocycle moiety of the optionally substituted heterocyclic group $R_{12}$ in the formula $R_{12}-\underset{\substack{\| \\ N-X-R_{13}}}{C}-$ (or $R_{10}$)

may be a 5-membered heterocyclic group containing one nitrogen, sulfur or oxygen atom with or without one more nitrogen atom. Examples of such heterocyclic group are 2-thiazolyl, 4-thiazolyl, 5-thiazolyl, 2-thienyl, 3-thienyl, 2-furyl, 3-furyl, 2-pyrrolyl and 3-pyrrolyl. The substituents on these heterocyclic groups are, for example, lower ($C_{1-3}$) alkyl, lower ($C_{1-3}$) alkoxy, hydroxyl, mesyl, halogen, imino, amino, mesylamino, and ($C_{2-4}$) alkylcarbonyl-amino which may be substituted by halogen.

The substituent moiety of the optionally substituted phenyl $R_{12}$ includes lower ($C_{1-3}$) alkyl, lower ($C_{1-3}$) alkoxy, halogen, nitro, amino, hydroxyl, substituted hydroxyl. The substituent in said substituted hydroxyl is, for example, benzyl, benzoyl, ($C_{2-10}$) alkylcarbonyl, γ-D-glutamyl or 3-amino-3-carboxypropyl.

The substituent moiety of the optionally substituted phenyl $R_{13}$ includes lower ($C_{1-3}$) alkyl, lower ($C_{1-3}$) alkoxy, halogen, etc.

With regard to the optionally substituted lower alkyl-carbonyl $R_{13}$, the lower alkylcarbonyl preferably contains 2-4 carbon atoms, and the substituent is, for example, halogen.

The lower alkyl $R_{13}$ preferably contains 1 to 3 carbon atoms.

The lower alkylene $R_{14}$ in the formula $-R_{14}-R_{15}$ (i.e. $R_{13}$) preferably contains 1-3 carbon atoms, and is for example methylene, ethylene, methylethylene, dimethylethylene and ethylmethylene.

The lower alkenylene $R_{14}$ preferably contains 1-3 carbon atoms, and is for example vinylene or propenylene.

As examples of the ester moiety of carboxylic acid ester $R_{15}$, there may be mentioned methyl, ethyl, propyl, t-butyl and p-nitrobenzyl esters.

The heterocyclic group $R_{15}$ may be a 6-membered one containing one nitrogen atom and one oxygen atom. Mor-pholino is an example.

The lower alkyl $R_{16}$ in the formula $-CO-NH-\underset{\displaystyle R_{16}}{\underset{|}{C}H}-$ (or $R_{11}$) preferably contains 1-3 carbon atoms.

The substituent in the optionally substituted phenyl $R_{16}$ includes lower ($C_{1-3}$) alkyl, lower ($C_{1-3}$) alkoxy, halogen, nitro, amino, ($C_{2-10}$) alkylcarbonyl, etc.

The heterocyclic group $R_{16}$ which may be substituted is, for example, 5-membered heterocyclic groups containing one sulfur, nitrogen or oxygen atom, 5-membered heterocyclic groups containing 1-2 nitrogen atoms and one sulfur or oxygen atom, or 5-membered heterocyclic groups containing 2-4 nitrogen atoms. Examples of such heterocyclic group are thiazolyl, isothiazolyl, oxazolyl, isoxazolyl, thienyl, furyl, pyrrolyl, thiadiazolyl, oxadiazolyl, triazinyl, tetrazolyl, imidazolyl, pyrazinyl, pyrimidinyl, pyridazinyl and piperazinyl. The substituents on these include lower ($C_{1-3}$) alkyl, lower ($C_{1-3}$) alkoxy, halogen, hydroxyl, amino, ($C_{2-4}$) alkylcarbonylamino which may be substituted by halogen.

The substituent in the optionally substituted sulfamoyl $R_{17}$ is, for example, lower ($C_{1-3}$) alkyl or amidino.

The substituent in the optionally substituted phenoxycarbonyl $R_{17}$ is, for example, lower ($C_{1-3}$) alkyl or lower ($C_{1-3}$) alkoxy.

The lower alkyl or lower alkoxy $R_{18}$ preferably contains 1-3 carbon atoms.

The substituent in the optionally substituted phenyl $R_{19}$ is, for example, lower ($C_{1-3}$) alkyl, lower ($C_{1-3}$) alkoxy, halogen, nitro, amino, hydroxyl or substituted aminomethyl. The substituent in said substituted aminomethyl includes among others carbamoyl, (2-oxo-3-benzylideneaminoimidazolidin-1-yl)carbonyl and (2-oxoimidazolidin-1-yl)carbonyl.

The substituent in the optionally substituted phenoxy $R_{19}$ is, for example, lower ($C_{1-3}$) alkyl, lower ($C_{1-3}$) alkoxy, halogen, nitro, amino, hydroxyl or aminomethyl.

The substituent is as mentioned above for the substituent on the optionally substituted phenyl $R_{19}$. The optionally substituted lower alkyl $R_{19}$ preferably contains 1-6 carbon atoms, and the substituent is, for example, halogen, hydroxyl, cyano or trifluoromethyl.

The alkenylene in the optionally substituted alkenylene $R_{19}$ is, for example, vinylene or propenylene, and the substituent is, for example, carboxyl or cyano.

The heterocyclic ring in the optionally substituted heterocyclic group $R_{19}$ may be 5- or 6-membered heterocyclic groups containing one sulfur atom or 1-4 nitrogen atoms, or 5- or 6-membered heterocyclic groups containing one sulfur atom and one nitrogen or oxygen atom. Examples of the heterocyclic group are 2-thienyl, benzothienyl, 3-thienyl, 2-pyridyl, 3-pyridyl, 4-pyridyl, 2-thiazolyl, 4-thiazolyl, 5-thiazolyl, isothiazolyl, 1-tetrazolyl, 5-tetrazolyl, pyrrolidinyl, imidazolyl and 1,4-oxathiin.

The substituent in the optionally substituted heterocyclic group $R_{19}$ is, for example, lower $(C_{1-3})$ alkyl, lower $(C_{1-3})$ alkoxy, halogen, nitro, hydroxyl, optionally protected amino, carboxyl, oxo, $(C_{2-4})$ alkylcarbonylamino which may be substituted by halogen, or $(C_{2-4})$ alkylcarbonyl.

Among the terms as used hereinabove in relation to the acyl groups or moieties, the $(C_{1-12})$ alkyl includes, among others, methyl, trifluoromethyl, ethyl, n-propyl, isopropyl, n-butyl, isobutyl, sec-butyl, tert-butyl, pentyl, isopentyl, hexyl, isohexyl, heptyl, 3-heptyl, octyl, nonyl, decyl, undecyl, dodecyl and cyclohexyl. The lower $(C_{1-6})$ alkyl includes, among others, methyl, trifluoromethyl, ethyl, n-propyl, isopropyl, n-butyl, isobutyl, sec-butyl, tert-butyl, pentyl, isopentyl, hexyl and isohexyl. The $(C_{1-3})$ lower alkyl includes, among others, methyl, trifluoromethyl, ethyl, n-propyl and isopropyl. The lower $(C_{1-3})$ alkoxy includes methoxy, ethoxy, n-propoxy and isopropoxy.

Examples of the halogen mentioned in relation to the

above formulas are chlorine, bromine, iodine and fluorine.

Examples of the lower ($C_{1-3}$) alkylsulfonyl are methylsulfonyl, ethylsulfonyl, n-propylsulfonyl and isopropylsulfonyl.

Examples of the ($C_{2-4}$) alkylcarbonylamino are acetylamino, propionylamino, n-butyrylamino and isobutyrylamino. Examples of the ($C_{2-10}$) alkylcarbonyloxy are acetoxy, n-propionyloxy, n-butyryloxy, isobutyryloxy, n-pentanoyloxy, n-hexanoyloxy, n-heptanoyloxy, n-octanoyloxy, n-nonanoyloxy and n-decanoyloxy.

The number of said substituents may be preferably one to three.

Relative to the above-mentioned acyl groups, examples of the acyl of the formula $R_6$-CO- ($R_6$ being as above defined) are [3-(2,6-dichlorophenyl)-5-methylisoxazol-4-yl]-carbonyl, 4-ethyl-2,3-dioxo-1-piperazinecarbonyl and (2-oxoimidazolidin-1-yl)carbonyl.

Example of the acyl group of the formula $R_7-NH-\underset{R_9}{\underset{|}{C}H}-CO-$ ($R_7$ and $R_9$ being as above defined) are D-alanyl, D-phenylalanyl, α-benzyl-N-carbobenzoxy-γ-D-gultamyl-D-alanyl, D-phenylglycyl-D-alanyl, N-carbobenzoxy-D-phenylglycyl, D-alanyl-D-phenylglycyl, γ-D-glutamyl-D-alanyl, N-carbobenzoxy-D-alanyl-D-phenylglycyl, D-carbamoyltriptophyl-D-phenylglycyl, N-[2-amino-3-(N-methylcarbamoyl)-propionyl]-D-phenylglycyl, N-[2-carbobenzoxyamino-3-(N-methylcarbamoyl)propionyl]-D-phenylglycyl, N-carbobenzoxy-D-phenylglycyl-D-phenylglycyl, 2-(2,3-diaminopropionamido)-2-phenylacetyl, D-alanyl-D-alanyl, 2-[2-amino-3-(N-methylcarbamoyl)propionamido]acetyl, 2-(2-amino-3-sulfamoylpropionamido)-2-phenylacetyl, 2-[2-amino-3-(4-ethyl-2,3-dioxo-1-piperazinecarboxamido)propionamido]-2-phenylacetyl, D-2-(4-ethyl-2,3-dioxo-1-piperazinecarboxamido)-2-(4-methoxyphenyl)acetyl, D-2-[2-(4-ethyl-2,3-dioxo-1-piperazinecarboxamido)-3-(N-methylcarbamoyl)propionamido]-2-phenylacetyl, D-2-[2-(4-ethyl-2,3-dioxo-1-piperazinecarboxamido)-acetamido]-2-phenylacetyl, D-2-(3-sulfamoyl-2-benzyloxycarboxamidopropionamido)-2-phenylacetyl, D-2-[2-benzyloxy-

carboxamido-3-(4-methoxyphenyloxycarboxamido)propionamido]-2-phenylacetyl, 2-[2-benzyloxycarboxamido-3-(N-methylcarbamoyl)propionamido]acetyl, 2-(N-carbobenzoxy-D-phenyl-glycylamino)-3-(N-methylcarbamoyl)propionyl, N-carbobenzoxy-D-alanyl, 2-benzyloxycarboxamido-3-(N-methylcarbamoyl)-propionyl, D-2-[2-(4-ethyl-2,3-dithioxo-1-piperazinecarbox-amido)-2-phenylacetyl, 2-(2-aminothiazol-4-yl)-2-(4-ethyl-2,3-dioxo-1-piperazinecarboxamido)acetyl, 2-(2-phenyl-acetamido)propionyl, 2-(4-ethyl-2,3-dioxo-1-piperazinecarboxamido)-2-thienylacetyl, D-2-(4-n-dodecyl-2,3-dioxo-1-piperazinecarboxamido)-2-phenylacetyl, D-2-(4,6-diethyl-2,3-dioxo-1-piperazinecarboxamido)-2-phenylacetyl, D-2-(4-cyclohexyl-2,3-dioxo-1-piperazinecarboxamido)-2-phenylacetyl, D-2-(4-cyclohexyl-2,3-dioxo-1-piperazinecarboxamido)-2-thienylacetyl, D-2-(4-n-amyl-6(S)-methyl-2,3-dioxo-1-piperazinecarboxamido)-2-thienylacetyl, D-2-(4-ethyl-5(R)-methyl-2,3-dioxo-1-piperazinecarboxamido)-2-thienylacetyl, 2-(8-hydroxy-1,5-naphthyridine-7-carboxamido)-2-phenylacetyl, 2-(4-n-octyl-2,3-dioxo-1-piperazinecarboxamido)-2-phenylacetyl, 2-(4-ethyl-2,3-dioxo-1-piperazinecarbox-amido)-2-(4-hydroxysulfonyloxyphenyl)acetyl, 2-(4-ethyl-2,3-dioxo-1-piperazinecarboxamido)-2-(4-chlorophenyl)acetyl, 2-(4-n-octyl-2,3-dioxo-1-piperazinecarboxamido)-2-(4-hydroxysulfonyloxyphenyl)acetyl, 2-(4-ethyl-2,3-dioxo-1-piperazinecarboxamido)-2-(4-trimethylsilylphenyl)acetyl, 2-(4-ethyl-2,3-dioxo-1-piperazinecarboxamido)-2-(3-chloro-4-methoxyphenyl)acetyl, 2-(4-ethyl-2,3-dioxo-1-piperazine-carboxamido)-2-(3-chloro-4-hydroxysulfonyloxyphenyl)acetyl, 2-(4-ethyl-2,3-dioxo-1-piperazinecarboxamido)-2-(3-chloro-4-hydroxyphenyl)acetyl, 2-(4-ethyl-2,3-dioxo-1-piperazine-carbonyl)-2-(4-benzyloxyphenyl)acetyl, 2-(4-n-octyl-2,3-dioxo-1-piperazinecarboxamido)-2-(4-hydroxyphenyl)acetyl, α-[N-(4-ethyl-2,3-dioxo-1-piperazinecarbonyl)]glutaminyl, N-(4-ethyl-2,3-dioxo-1-piperazinecarbonyl)phenylalanyl, N-(4-ethyl-2,3-dioxo-1-piperazinecarbonyl)-D-alanyl, 2-(4-ethyl-2,3-dioxo-1-piperazinecarboxamido)-2-(4-hydroxyphenyl)-acetyl, 2,2-bis(4-ethyl-2,3-dioxo-1-piperazinecarboxamido)-acetyl, 2-(4-ethyl-2,3-dioxo-1-piperazinecarboxamido)-2-(1-cyclohexen-1-yl)acetyl, 2-(4-n-octyl-2,3-dioxo-1-piperazinecarboxamido)-2-thienylacetyl, 2-(4-ethyl-2,3-dioxo-1-piperazinecarboxamido)-2-(2-chloroacetamidothiazol-4-yl)acetyl, 2-(4-ethyl-2,3-dioxo-1-piperazinecarboxamido)-2-(2-methylthiazol-4-yl)acetyl, 2-(4-ethyl-2,3-dioxo-1-piperazinecarboxamido)-2-(2-acetamidothiazol-4-yl)acetyl, 2-(4-n-octyl-2,3-dioxo-1-piperazinecarboxamido)-2-(2-aminothiazol-4-yl)acetyl, 2-(4-ethyl-2,3-dioxo-1-piperazinecarboxamido)-2-furylacetyl, 2-(4-ethyl-2,3-dioxo-1-piperazinecarboxamido)-2-(2-pyrrolyl)acetyl, 2-(4-ethyl-2,3-dithioxo-1-piperazinecarboxamido)-2-(4-hydroxyphenyl)-acetyl, 2-(4-n-octyl-2,3-dioxo-1-piperazinecarboxamido)-2-(2-chloroacetamidothiazol-4-yl)acetyl, N-(4-ethyl-2,3-dioxo-1-piperazinecarbonyl)-D-methionyl, D-2-[4-(2-phenylethyl)-2,3-dioxo-1-piperazinecarboxamido]phenylacetyl, D-2-(4-ethyl-2,3-dioxo-1-piperazinecarboxamido)-2-(4-benzoyloxyphenyl)acetyl, 2,5-bis(4-ethyl-2,3-dioxo-1-piperazinecarboxamido)pentanoyl, 2-(4-ethyl-2,3-dioxo-1-piperazinecarboxamido)-3-(N-methylcarbamoyl)propionyl, 2,3-bis(4-ethyl-2,3-dioxo-1-piperazinecarboxamido)propionyl, 2-(4-ethyl-2,3-dioxo-1-piperazinecarboxamido)-3-chloropropionyl, 2-(4-ethyl-2,3-dioxo-1-piperazinecarboxamido)-2-(4-n-octanoyloxyphenyl)acetyl, 2-(4-ethyl-2,3-dioxo-1-piperazinecarboxamido)-3-sulfamoylpropionyl, 3-[2-(4-ethyl-2,3-dioxo-1-piperazinecarboxamido)-3-[(1-methyl-1H-tetrazol-5-yl)-thio]propionyl, 2-(4-ethyl-2,3-dioxo-1-piperazinecarboxamido)-acetyl, D-2-[4-(2-hydroxyethyl)-2,3-dioxo-1-piperazinecarboxamido]-2-phenylacetyl, D-2-[4-(2-chloroethyl)-2,3-dioxo-1-piperazinecarboxamido]-2-phenylacetyl, 2-(4-ethyl-2,3-dioxo-1-piperadinecarboxamido)-3-(ethoxycarbonylmethylcarbamoyl)propionyl, 2-(4-ethyl-2,3-dioxo-1-piperazinecarboxamido)-3-(thienylacetamido)propionyl, 2-(4-ethyl-2,3-dioxo-1-piperazinecarboxamido)-3-[2-(1H-tetrazol-1-yl)-acetamido]propionyl, 2-(4-ethyl-2,3-dioxo-1-piperazinecarbox-

amido)-2-(1H-tetrazol-1-yl)acetyl, 2-[(3-furfurylideneamino-2-oxoimidazolidin-1-yl)carboxamido]-2-phenylacetyl, 2-[3- furfurylideneamino-2-oxoimidazolidin-1-yl)carboxamido]-2-(4-hydroxysulfonyloxyphenyl)acetyl, 2-[[2-oxo-3-(thiophene-2-aldoimino)imidazolidin-1-yl]carboxamido]-2-phenylacetyl, 2-[(3-furfurylideneamino-2-oxoimidazolidin-1-yl)carboxamido]-2-thienylacetyl, D-2-[(3-methylsulfonyl-2-oxoimidazolidin-1-yl)carboxamido]-2-phenylacetyl, 2-[(3-furfurylideneamino-2-oxoimidazolidin-1-yl)carboxamido]-2-(2-aminothiazol-4-yl)acetyl, 2-[(3-furfurylideneamino-2-oxoimidazolidine-1-yl)carboxamido]-2-(2-chloroacetamido-thiazol-4-yl)acetyl, 2-[[2-oxo-3-(thiophene-2-aldoimino)-imidazolidin-1-yl]carboxamido]-2-thienylacetyl, 2-[(3-mesyl-2-oxoimidazolidin-1-yl)carboxamido]-2-thienylacetyl, D-2-[(3-furfurylideneamino-2-oxoimidazolidin-1-yl)-carboxamido]propionyl, 2-(4-hydroxy-6-methylnicotinamido)-2-phenylacetyl, 2-(4-hydroxy-6-methylnicotinamido)-2-(4-hydroxyphenyl)acetyl, 2-[5,8-dihydro-2-(4-formyl-1-piperazinyl)-5-oxopyrido[2,3-d]pyrimidine-6-carboxamido]-2-phenylacetyl, 2-(3,5-dioxo-1,2,4-triazine-6-carboxamido)-2-(4-hydroxyphenyl)acetyl, D-3-[(2-oxo-3-sulfoimidazolidin-1-yl)carboxamido]-2-thienylacetyl, D-2-[(5-methoxycarbonyl-3-methyl-2-oxoimidazolidin-1-yl)carboxamido]-2-phenyl-acetyl, D-2-[(5-benzyloxycarbonyl-3-methyl-2-oxoimidazolidin-1-yl)carboxamido]-2-phenylacetyl, D-2-[(5-carboxyl-3-methyl-2-oxoimidazolidin-1-yl)carboxamido]-2-phenylacetyl, 2-(coumarin-3-carboxamido)-2-phenylacetyl, 2-(4-ethyl-2,3-dioxo-1-piperazinecarboxamido)-2-(2-chloro-1-cyclohexen-1-yl)acetyl, D-2-(4-n-amyl-2,3-dioxo-1-piperazinecarbox-amido)-2-phenylacetyl, D-2-(4-n-amyl-6(R)-methyl-2,3-dioxo-1-piperazinecarboxamido)-2-thienylacetyl, 2-(4-hydroxy-7-methyl-1,8-naphthyridine-3-carboxamido)-2-phenylacetyl, 2-(4-hydroxy-7-trifluoromethylquinoline-3-carboxamido)-2-phenylacetyl, N-[2-(2-aminothiazol-4-yl)-acetyl]-D-phenylglycyl, 2-(6-bromo-1-ethyl-1,4-dihydro-4-oxothieno[2,3-b]pyridine-3-carboxamido)-2-phenylacetyl,

2-[2-(2-chloroacetamidothiazol-4-yl)acetamido]-2-phenylacetyl, 2-(3,5-dioxo-1,2,4-triazino-6-carboxamido)-2-thienylacetyl, 2-(2,4-dioxopyrimidino-5-carboxamido)-2-thienylacetyl, 2-[2-(4-ethyl-2,3-dioxo-1-piperazinecarboxamido)-2-thienylacetamido]-2-phenylacetyl, 2-(2-ureido-2-thienylacetamido)-2-phenylacetyl, 2-(2-ureido-2-thienylacetamido)-2-(4-hydroxysulfonyloxyphenyl)acetyl, 2-(2-ureido-2-thienylacetamido)-2-(4-hydroxyphenyl)acetyl, 2-(N-carbobenzoxyprolylamino)-2-furylacetyl, α-(thienylmethylcarbonyl)alanyl, 2-(4-chlorobenzoylureido)-2-thienylacetyl, 2-(2-thienylacetamido)acetyl, N-benzyloxycarboxamido-D-alanyl, N-(4-hydroxybenzoyl)-D-alanyl, 2-(4-chlorobenzamido)propionyl, 2-(4-aminobenzamido)acetyl, N-(4-ethyl-2,3-dioxo-1-piperazinecarbonyl)methionyl-D-phenylglycyl, D-2-[2-(2,6-dichlorophenylthio)acetamido]-2-phenylacetyl, D-2-[[3-(2,6-dichlorophenyl)-5-methylisoxazol-4-yl]carboxamido]-2-thienylacetyl, 2-ureido-2-thienylacetyl, N-carbamoyl-D-phenylglycyl, 2-(3-methylcarbamoyl-3-methyl-1-ureido)-2-phenylacetyl, 2-(3-methylcarbamoyl-3-methyl-1-ureido)-2-(4-hydroxyphenyl)-acetyl, 2-(3-methylcarbamoyl-3-methyl-1-ureido)-2-thienylacetyl, 2-[3-(2-hydroxybenzoyl)-1-ureido]-2-phenylacetyl, 2-[3-(2-benzyloxybenzoyl)-1-ureido-2-(4-hydroxysulfonyloxyphenyl)acetyl, 2-[3-(2-hydroxybenzoyl)-1-ureido]-2-(4-hydroxyphenyl)acetyl, 2-[3-(2-benzyloxybenzoyl)-1-ureido]-2-phenylacetyl, 2-[3-(2-benzyloxybenzoyl)-1-ureido]-2-(4-hydroxyphenyl)acetyl, D-2-[2-(benzyloxycarboxamido)-2-(benzyloxycarbonyl)ethanesulfonamido]-2-phenylacetyl, N-methyl-D-phenylglycyl, etc.

Examples of the acyl group of the formula $R_{10}$-$R_{11}$-CO- ($R_{10}$ and $R_{11}$ being as above defined) are N-[2-(2-aminothiazol-4-yl)-2-methoxyiminoacetyl]-D-alanyl, N-[2-(2-aminothiazol-4-yl)-2-methoxyiminoacetyl]-D-phenylglycyl, 2-(2-aminothiazol-4-yl)-2-[2-(2-aminothiazol-4-yl)-2-methoxyiminoacetamido]acetyl, 2-(2-chloroacetamidothiazol-4-yl)-2-[2-(2-chloroacetamidothiazol-4-yl)-2-methoxyimino-acetamido]acetyl, 2-(2-chloroacetamidothiazol-4-yl)-2-methoxyiminoacetyl, 2-(2-aminothiazol-4-yl)-2-isopropoxy-iminoacetyl, 2-(2-aminothiazol-4-yl)-2-methoxyiminoacetyl, 2-(2-aminothiazol-4-yl)-2-oxyiminoacetyl, 2-thienyl-2-methoxyiminoacetyl, 2-furyl-2-methoxyiminoacetyl, 2-(4-hydroxyphenyl)-2-methoxyiminoacetyl, 2-phenyl-2-methoxyiminoacetyl, 2-phenyl-2-oxyiminoacetyl, 2-thienyl-2-oxyiminoacetyl, 2-thienyl-2-(dichloroacetyloxyimino)acetyl, 2-[4-(γ-D-glutamyloxy)phenyl]-2-oxyiminoacetyl, 2-[4-(3-amino-3-carboxypropoxy)phenyl]-2-oxyiminoacetyl, 2-thienyl-2-(3-morpholinopropyloxyimino)acetyl, 2-(5-chloro-2-chloro-acetamidothiazol-4-yl)-2-methoxyiminoacetyl, 2-(5-chloro-2-aminothiazol-4-yl)-2-methoxyiminoacetyl, 2-[1-(t-butoxy-carbonyl)isopropoxyimino]-2-(2-sulfoaminothiazol-4-yl)-acetyl, 2-[1-(t-butoxycarbonyl)isopropoxyimino]-2-(2-triphenylmethylaminothiazol-4-yl)acetyl, 2-(2-chloroacet-amidothiazol-4-yl)-2-isopropoxyiminoacetyl, 2-methoxyimino-2-(2-sulfoaminothiazol-4-yl)acetyl, 2-[2-(2-chloroacetamido-thiazol-4-yl)-2-methoxyiminoacetamido]-2-phenylacetyl, 2-phenyl-2-p-tolylthioimionacetyl, 2-[2-(2-aminothiazol-4-yl)-2-methoxyiminoacetamido]acetyl, 2-(2-mesylaminothiazol-4-yl)-2-isopropoxyiminoacetyl, 2-(2-imino-3-mesyl-4-thiazolin-4-yl)-2-isopropoxyiminoacetyl, etc.

Examples of the acyl group of the formula 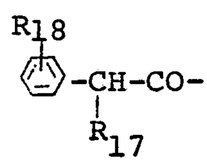

($R_{17}$ and $R_{18}$ being as above defined) are α-sulfophenyl-acetyl, α-hydroxysulfonyloxyphenylacetyl, α-hydroxyphenyl-acetyl, α-sulfamoylphenylacetyl, α-(phenoxycarbonyl)phenyl-acetyl, α-(p-tolyloxycarbonyl)phenylacetyl, α-formyloxy-phenylacetyl, α-carboxyphenylacetyl, α-benzyloxycarbonyl-phenylacetyl, 2-(N,N-dimethylsulfamoyl)-2-phenylacetyl, 2-bromo-2-phenylacetyl, 2-azido-2-phenylacetyl, 2-phthalimido-2-thienylacetyl, 2-azido-2-(3-chlorophenyl)-acetyl, etc.

Examples of the acyl group of the formula $R_{19}$-$R_{20}$-

$CH_2$-CO-($R_{19}$ and $R_{20}$ being as above defined) are cyanoacetyl, phenylacetyl, phenoxyacetyl, trifluoromethylthioacetyl, cyanomethylthioacetyl, 1H-tetrazolyl-1-acetyl, 2-thienylacetyl, 2-(2-aminothiazol-4-yl)acetyl, 2-(2-chloroacetamidothiazol-4-yl)acetyl, 4-pyridylthioacetyl, 2-thienylthioacetyl, 3,5-dichloro-1,4-dihydro-4-oxopyridine-1-acetyl, β-carboxyvinylthioacetyl, 2-(2-aminomethylphenyl)acetyl, 2-(2-N-carbobenzoxyaminomethylphenyl)acetyl, 2-(2-ureidomethylphenyl)acetyl, 2-[2-[(2-oxoimidazolidin-1-yl)carbonylaminomethyl]phenyl]acetyl, 2-[2-[(3-benzylideneamino-2-oxoimidazolidin-1-yl)carbonylaminomethyl]phenyl]acetyl, 2-(5,6-dihydro-1,4-oxathin-2-yl)-acetyl, 2-(2,5-dioxopyrrolidin-3-yl)acetyl, 2-succinimidoacetyl, 2-(1-acetyl-2,4-dioxoimidazolidin-3-yl)acetyl, etc.

The amino and/or carboxyl group in the above acyl groups may be protected.

The protective groups for said carboxyl moiety of the acyl group include all groups generally usable as carboxyl-protecting groups in the field of β-lactam compounds and organic chemistry, their ester moieties being, for example, methyl, ethyl, propyl, isopropyl, t-butyl, t-amyl, benzyl, p-nitrobenzyl, p-methoxybenzyl, benzhydryl, phenacyl, phenyl, p-nitrophenyl, methoxymethyl, ethoxymethyl, benzyloxymethyl, acetoxymethyl, pivaloyloxymethyl, β-methylsulfonylethyl, methylthiomethyl, trityl, β,β,β-trichloroethyl, β-iodoethyl, t-butyldiphenylsilyl, 2-trimethylsilylethyl, 2-cyanoethyl, trimethylsilyl, dimethylsilyl, acetylmethyl, p-nitrobenzoylmethyl, p-mesylbenzoylmethyl, pnthalimidomethyl, propionyloxymethyl, 1,1-dimethylpropyl, 3-methyl-3-butenyl, succinimidomethyl, 3,5-di-tert-butyl-4-hydroxybenzyl, mesylmethyl, benzenesulfonylmethyl, phenylthiomethyl, dimethylaminoethyl, pyridine-1-oxide-2-methyl, methylsulfinylmethyl, bis(p-methoxyphenyl)methyl, 2-cyano-1,1-dimethylethyl, and silyl ester groups. The object of the present invention is to provide the above-mentioned novel monocyclic compounds, and therefore no restrictions or limitations are posed in selecting the

protective group. However, benzyl, β,β,β-trichloroethyl, benzhydryl, 2-trimethylsilylethyl, t-butyl, p-nitrobenzyl and p-methoxybenzyl are especially preferable.

The amino-protecting groups in the above formulas (I) and (II) are conveniently those that are in use in the field of β-lactam and peptide syntheses. They are, for example, aromatic acyl groups such as phthaloyl, p-nitrobenzoyl, p-tert-butylbenzoyl, p-tert-butylbenzenesulfonyl, benzenesulfonyl and toluenesulfonyl, aliphatic acyl groups such as formyl, acetyl, propionyl, monochloroacetyl, dichloroacetyl, trichloroacetyl, methanesulfonyl, ethane sulfonyl, trifluoroacetyl, maloyl and succinyl, esterified carboxyl groups such as methoxycarbonyl, ethoxycarbonyl, t-butoxycarbonyl, isopropoxycarbonyl, 2-cyanoethoxycarbonyl, β,β,β-trichloroethoxycarbonyl, benzyloxycarbonyl, p-nitrobenzyloxycarbonyl, p-methoxybenzyloxycarbonyl, 2-trimethylsilylethoxycarbonyl, diphenylmethyloxycarbonyl, methoxymethyloxycarbonyl, acetylmethyloxycarbonyl, isobornyloxycarbonyl and phenyloxycarbonyl, methylene groups such as (hexahydro-1H-azepin-1-yl)methylene, and sulfonyl groups such as 2-amino-2-carboxyethylsulfonyl, and further non-acyl amino-protecting groups such as trityl, 2-nitrophenylthio, benzylidene, 4-nitrobenzylidene, di- or trialkylsilyl, benzyl and p-nitrobenzyl. The selection of said protective group is not critical in this invention as in the case of the carboxyl-protecting group. Nevertheless, monochloroacetyl, benzyloxycarbonyl, 2-trimethylsilylethoxycarbonyl, p-methoxybenzyloxycarbonyl and p-nitrobenzyloxycarbonyl are especially preferred.

Furthermore, as the protective groups for the amino moiety of the carboxyl group, those described in "Protective Groups in Organic Chemistry"; compiled by McOmie, Plenum Press, N.Y. 1973 can be used likewise.

Among the optionally acylated or protected amino groups represented by $R_1$ and acylated or protected amino groups represented by $R_2$ in the above formulas, those

adequate for better antibacterial and β-lactamase inhibitory activities may be represented by the formula:

A-B-CONH-

wherein A is hydrogen, a lower alkyl group, an alicyclic group such as cyclohexyl or cyclohexenyl, an aryl group such as phenyl, an aralkyl group such as benzyl, or a heterocyclic group such as thienyl, benzothienyl, pyrrolyl, isoxazolyl, piperazinyl, thiazolyl, tetrazolyl or oxathiano, and such A may have one or two substituents such as amino, lower alkyl, lower alkoxy, phenyl, 2,6-dichlorophenyl, oxo, hydroxyl, halogen or chloroacetamido, B is (1) a group represented by tye formula:

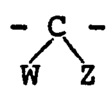

wherein Z is hydrogen and W is an optionally esterified carboxyl group, a sulfo group, a sulfamoyl group, a hydroxysulfonyloxy group, an optionally protected amino group, an amido group such as an arylcarboxamido (e.g. phenylcarboxamido) or lower alkylcarboxamido group, or a heterocyclecarboxamido group such as 2,3-dioxo-1-piperazinecarboxamido, imidazolidinocarboxamido, oxoimidazolidinecarboxamido, (isoxazol-4-yl)carboxamido, (2-aminothiazol-4-yl)methylcarboxamido or 3-(2,3-dioxo-1-piperazinecarboxamido)-2-carbobenzoxyaminopropionamido, (2) a group of the formula =C=N-X'-G (in which X' is oxygen or sulfur atom or a sulfoxide group and G is a lower alkyl group, a carboxyl(lower)alkyl group such as α,α-dimethyl-α-carboxymethyl, an aryl group such as phenyl, or an acyl group such as acetyl), (3) a single bond or (4) $-\overset{O}{\overset{\|}{C}}-$.

In the above formula, the lower alkyl group A is preferably a straight or branched chain alkyl group containing 1-4 carbon atoms, may be exemplified as methyl, ethyl and iso-butyl, and may have, as a substituent other than such one as mentioned above, N-methylcarbamoyl, carbobenzyloxyamino, an aryl group such as phenyl, tetrazolylacetamido, 4-ethyl-2,3-dioxo-1-piperazinecarboxamido,

or a heterocyclic group such as 1,2-diazole which may have phenyl, methyl or ethyl at the 3-position thereof. The lower alkyl as an optional substituted on A includes methyl and ethyl, the lower alkoxy as an optional substituent on A includes methoxy and ethoxy, and the halogen as an optional substituent on A includes fluorine, chlorine and bromine. The optionally protected amino group W includes chloroacetylamino, aralkylamino and aralkyloxycarbonylamino.

The lower alkyl moiety of the lower alkylcarboxamido group W is preferably a straight or branched alkyl group containing 1-4 carbon atoms, the lower alkyl moiety includes methyl, ethyl, propyl, isopropyl, butyl, iso-butyl, and the lower alkyl moiety may be substituted by a halogen atom such as chlorine, bromine or fluorine, sulfamoyl group or an optionally protected amino group being the same as above and the sulfamoyl group may be substituted by sulfo group.

The heterocycle moiety of the heterocycle-carboxamido group W may have one or two substituents exemplified by phenyl, a $C_{1-12}$ alkyl group (e.g. methyl, ethyl, n-propyl, iso-propyl, n-butyl, iso-butyl, sec-butyl, tert-butyl, pentyl, iso-pentyl, hexyl, iso-hexyl, heptyl, 3-heptyl, octyl, nonyl, decyl, undecyl, dodecyl), a saturated alicyclic group (e.g. cyclohexyl, cyclopentyl), a $C_{2-8}$ alkenyl group (e.g. vinyl, allyl, 2-butenyl, 4-methyl-3-pentenyl), an arylcarbonyl group (e.g. phenylcarbonyl) optionally substituted by a lower alkoxy group (e.g. methoxy, ethoxy), furfurylideneamino, thiophenealdoimino, sulfo, an alkoxycarbonyl group (e.g. methoxycarbonyl, ethoxycarbonyl), aralkyloxycarbonyl group (e.g. benzyloxycarbonyl) or carboxyl group.

In the present process, the compound (II) is reacted with t-butyl or isopropylsilyl compound. As the t-butyl or isopropylsilyl compound, there may be mentioned a compound of the formula:

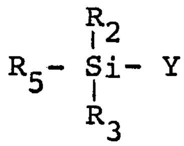 , (IV)

wherein $R_3$ and $R_4$ respectively stand for lower ($C_{1-4}$) alkyl, phenyl, benzyl, lower ($C_{1-4}$) alkoxy, $R_5$ stands for t-butyl or isopropyl, and Y stands for a reactive group to be liberated from the silyl compound.

As the lower alkyl represented by $R_3$ and $R_4$, there may be mentioned methyl, ethyl, n-propyl, isopropyl, n-butyl and t-butyl, and as the lower alkoxy represented by $R_3$ and $R_4$, there may be mentioned methoxy, ethoxy, n-propoxy, isopropoxy, n-butoxy and t-butoxy.

As the reactive group to be liberated from the silylating agent may be exemplified halogen (e.g. chloro, bromo), N-(trialkylsilyl)trifluoroacetimidoyloxy group, N-(trialkylsilyl)acetimidoyloxy group, halogeno, an acylamino group such as formylamino, acetylamino, propionylamino, butylylamino or trifluoroacetylamino, a (trialkylsilyl)amino group such as (tri-t-butyldimethylsilyl)amino, isopropyldimethylsilylamino or (chloromethyldimethylsilyl)amino, amino, an alkylamino group such as methylamino, ethylamino or propylamino, an N,N-dialkylamino group such as N,N-dimethylamino, N-chloromethyl-N-dimethylamino, N,N-diethylamino, N,N-dipropylamino, N-methyl-N-ethylamino, N-methyl-N-propylamino or N-ethyl-N-propylamino, or a heterocyclic group such as imidazolyl. As said alkyl, ones having 1 to 4 carbon atoms are preferable, and it is exemplified by methyl, ethyl, n-propyl, isopropyl, n-butyl and t-butyl.

Specific examples of the silyl compounds (IV) as described above, there may be mentioned N,O-bis(t-butyldimethylsilyl)trifluoroacetamide, N,O-bis(isopropyldimethylsilyl)acetamide, bis(dimethylisopropylsilyl)acetamide, isopropyldimethylsilylacetamide, bis(dimethyl-tert-butylsilyl)acetamide, N-methyl-N-t-butyldimethylsilylacetamide, N-methyl-N-isopropyldimethylsilyltrifluoroacetamide, N-t-

butyldimethylsilyldiethylamine, 1,3-bis(chloromethyl)-1,1,3,3-tetra-t-butyldimethyldisilazane, N-isopropyldimethylsilylimidazole, t-butyldiphenylchlorosilane, isopropyldiethylchlorosilane, isopropylmethyldichlorosilane, tert-butyldimethylchlorosilane, isopropyldimethylchlorosilane, or t-butyldiethylchlorosilane, Among them, tert-butyldimethylchlorosilane or isopropyldimethylchlorosilane is preferable. The present silylating reaction is followed by a *per se* conventional one. The reaction temperature of the silylation is in the range of about 0° to 50°C, preferably not higher than 38°C, usually a room temperature (about 20°C), and the reaction time is from several minutes (about 10 minutes) to about 24 hours. The reaction is conducted conveniently in, for example, ethyl acetate, dioxane, tetrahydrofuran, N,N-dimethylacetamide, N,N-dimethylformamide, dichloromethane, chloroform, benzene, toluene, acetone, methylethylketone, or acetonitrile, or an optional mixture of them, or any other solvent which is inert to this reaction. This reaction can be conducted also in the presence of an inorganic base such as sodium hydroxide, potassium hydroxide, sodium hydrogen carbonate, sodium carbonate or potassium carbonate or a trialkylamine such as triethylamine, tributylamine, tribenzylamine, N-methylmorpholine, or N-methylpiperidine, an organic tertiary amine such as N,N-dialkylaniline, N,N-dialkylbenzylamine, pyridine, picoline or lutidine, or an organic base such 1,5-diazabicyclo[2,2,2]-octane or 1,8-diazabicyclo[5,4,4]undecene-7, and when the base is liquid, it can be used also as a solvent.

Thus obtained 1-t-butyl or isopropylsilyl derivative of a compound (II), where $R_2$ is a protected amino group, can be led to a 1-t-butyl or isopropylsilyl derivative of a desired compound (II) by eliminating the protective group, followed by subjecting to acylation at 3-position. This acylation proceeds with good yield, and the process is simple and convenient, thus being very useful for the synthesis of various types of 2-oxoazetidine having a

desired acyl group as the substituent. Further, the sulfonation of the next step can be carried out continuously.

The elimination of amino-protective groups of the 1-t-butyl or isopropyl derivative of compounds (II) can be effected, according to the type of protective group, by selective application of per se known methods such as (1) a method involving the use of a base, (2) a method involving the use of hydrazine, (3) a method involving the use of thiourea or sodium N-methyldithiocarbamate or (4) a method involving reduction.

(1) The method involving the use of a base employs such an inorganic base as hydroxides, carbonates or metal alkoxides of alkali metals (e.g. sodium or potassium) or alkaline earth metals (e.g. calcium or magnesium), such an organic base as metal alkoxides (e.g. sodium methoxide, potassium methoxide, lithium methoxide, sodium ethoxide, potassium t-butoxide, lithium ethoxide, potassium ethoxide), organic amine (e.g. triethylamine, trimethylamine, tributylamine, N-methylmorpholine, N-methylpiperidine, N,N-dimethylaniline, pyridine, picoline, lutidine), or a quarternary ammonium salt (e.g. n-butylammonium hydroxide), and a basic ion exchange resin [e.g. Amberlite IRA-400 (Rohm and Haas Co., U.S.A.), Amberlite IR-45 (Rohm and Hass Co., U.S.A.), Dowex 1 (Dow Chemical Co., U.S.A.), Dowex 2 (Dow Chemical Co., U.S.A.)]. The method may be carried out in the presence of a solvent such as hydrophilic organic solvent (e.g. methanol, ethanol, propanol, tetrahydrofuran, dioxane, N,N-dimethylformamide, N,N-dimethylacetamide, dimethylsulfoxide, acetone, acetonitrile, nitromethane), water, or a mixed solvent thereof. The reaction temperature is normally about 0 to 30°C, preferably about 0 to 20°C.

(2) In the method involving the use of hydrazine, hydrazine or N-methylhydrazine is employed, and the reaction may be carried out in a solvent such as tetrahydrofuran, dioxane, diethylether, chloroform, dichloromethane, ester

(e.g. ethyl acetate), acetone, methanol, ethanol, benzene, toluene, N,N-dimethylformamide, N,N-dimethylacetamide, dimethylsulfoxide, and the reaction temperature is about 0° to 30°C, preferably 0° to 20°C, and the reaction time is about one to 24 hours.

(3) In the method involving the use of thiourea, it is carried out by employing, thiourea under mild reaction conditions so as to remove halogenoacetyl group [see Journal of the American Chemical Society 90, 4508 (1968); Journal of the Chemical Society 1915, 5015].

In the method involving the use of sodium N-methyldithiocarbamate, it is used about 1 equivalent to small excess amount of sodium N-methyldithiocarbamate relative to the starting material, the reaction is carried out in a solvent such as methanol, ethanol, propanol, diethylether, tetrahydrofuran, dioxane, chloroform, dichloromethane, and the reaction is carried out normally at about room temperature (about 20°C) and may be carried out under cooling (about -20°C) or under warming (about 40°C).

(4) The reductive method employs, according to the type of protective group and other conditions, a metal (e.g. tin, zinc, etc.) or a metal compound (e.g. chromous chloride, chromous acetate, etc.) together with an acid such as an organic acid (e.g. acetic acid or propionic acid, or involves the use of a metal catalyst for catalytic reduction. The catalyst used for such catalytic reduction may for example be platinum catalysts (e.g. platinum wire, platinum sponge, platinum black, platinum oxide, colloidal platinum,etc.), palladium catalysts (e.g. palladium sponge, palladium black, palladium oxide, palladium-barium sulfate, palladium-barium carbonate, palladium-carbon, palladium-silica gel, colloidal palladium, etc.), reduced nickel, nickel oxide, Raney nickel, Urushihara nickel, etc. The reductive method is usually conducted in a solvent. The method involving the use of a metal and an acid is usually carried out in the presence of water, acetone or the like.

In the catalytic reduction method, for instance, the reaction is conducted usually in the presence of an alcohol (e.g. methanol, ethanol, propanol, isopropyl alcohol, etc.), ethyl acetate, etc. The reaction temperature is not particularly critical, but may be selected suitably depending on the type of protecting groups or kinds of methods of eliminating the protective groups, and, preferably, mild conditions, i.e., ranging from under cooling (about 0°C) to under warming (about 40°C).

The compound (II), in which the amino group at 1-position is acylated, can be produced by subjecting thus-obtained 3-amino compound (i.e. 1-t-butyl or isopropyl derivative of the compound (III) wherein $R_2$ is amino group) to acylation by the use of a desired acylating agent, for example, an organic carboxylic acid containing an acyl group represented by $R_2$ or a reactive derivative thereof, for example, acid anhydrides, activated amides or activated esters described as follows:

1) Acid anhydrides:

The acid anhydrides include, among others, mixed anhydrides with a hydrogen halide (e.g. hydrochloric or hydrobromic acid), with a monoalkyl carbonate, with an aliphatic carboxylic acid (e.g. acetic, pivalic, valeric isopentanoic or trichloroacetic acid) or with an aromatic carboxylic acid (e.g. benzoic acid), and symmetric acid anhydrides.

2) Activated amides:

The activated amides include amides with pyrazole, imidazole, 4-substituted imidazole, dimethylpyrazole, benzotriazole, etc.

3) Activated esters:

The activated esters include, among others, such esters as methyl, ethyl, methoxymethyl, propargyl, 4-nitrophenyl, 2,4-dinitrophenyl, trichlorophenyl, pentachlorophenyl and mesylphenyl esters as well as esters of sucn acids as the above-mentioned carboxylic acid with

1-hydroxy-1H-2-pyridone, N-hydroxy-5-norbornene-2,3-dicarboximide, 1-hydroxybenzotriazole, N-hydroxysuccinimide and N-hydroxyphthalimide.

Appropriate reactive derivatives of organic acids are selected from among such ones as mentioned above depending on the type of the acid used. When a free acid is used as the acylating agent, the reaction is preferably carried out in the presence of a condensing agent. Examples of the condensing agent are N,N'-dicyclohexylcarbodiimide, N-cyclohexyl-N'-morpholinoethylcarbodiimide, N-cyclohexyl-N'-(4-diethylaminocyclohexyl)carbodiimide and N-ethyl-N'-(3-dimethylaminopropyl)carbodiimide, pentamethyleneketene-N-cyclohexylimine, trialkyl phosphite, thionyl chloride, oxalyl chloride, triphenylphosphine, N-ethylbenzoisoxazolium salt, or Vilsmeyer reagent.

The acylation is usually carried out in a solvent. The solvent includes, for example, water, acetone, dioxane, acetonitrile, methylene chloride, chloroform, dichloroethane, tetrahydrofuran, ethyl acetate, dimethylformamide, pyridine and other common organic solvents inert to the reaction. Among these, hydrophilic solvents may be used as mixtures with water.

The acylation may be carried out in the presence of an inorganic base, such as alkalimetal, or in the presence of an organic base such as an trialkylamine as trimethylamine, triethylamine, tributylamine, N-methylmorpholine or N-methylpiperidine or such an organic tertiary amine as N,N-dialkylaniline, N,N-dialkylbenzylamine, pyridine, picoline or lutidine, or such an organic base as 1,5-diazabicyclo[4,3,0]non-5-ene, 1,4-diazabicyclo[2,2,2]octane or 1,8-diazabicyclo[5,4,4]undecene-7. When the organic base or the above-mentioned condensing agent is a liquid, it may also serve as a solvent. The reaction temperature is not critical, but the reaction is mostly carried out under cooling (about 0°C) to about 30°C.

In acylation reaction the acylating agent contains

at least one asymmetric carbon atom, the respective stereoisomers alone as well as mixtures thereof can be subjected to the acylation. When the acylation product is a mixture of corresponding isomers, the individual isomers can be isolated as necessary by a conventional method, such as column chromatography or recrystallization.

The reaction step of this invention is to subject a 1-t-butyl or isopropylsilyl derivative of a compound (II) to sulfonation to give a 1-sulfo-2-oxoazetidine derivative (I), and this reaction is conducted by bringing a 1-t-butyl or isopropylsilyl derivative of the compound (II) into contact with sulfuric anhydride or a reactive derivative of sulfuric anhydride.

The reactive derivative of sulfuric anhydride is for example, sulfuric anhydride-pyridine, sulfuric anhydride-N,N-dimethylformamide, sulfuric anhydride-dioxane, sulfuric anhydride-trimethylamine or sulfuric anhydride-chlorosulfonic acid adduct.

In the above reaction, sulfuric anhydride or a reactive derivative thereof is added in an amount of about 1 to about 5 moles, preferably about 1 to about 2 moles, per mole of a 1-t-butyl or isopropylsilyl derivative of the compound (II).

The reaction temperature is about 0°C to about 80°C, preferably about 10°C to about 40°C. A solvent may be used in the above reaction. Usable solvents include ethers such as dioxane, tetrahydrofuran and diethyl ether, esters such as ethyl acetate and ethyl formate, halogenated hydrocarbons such as chloroform and methylene chloride, hydrocarbons such as benzene, toluene and n-hexane, amides such as dimethylformamide and dimethylacetamide, and other usual organic solvents, alone or in combination.

It is believed that the present sulfonation proceeds through the following route:

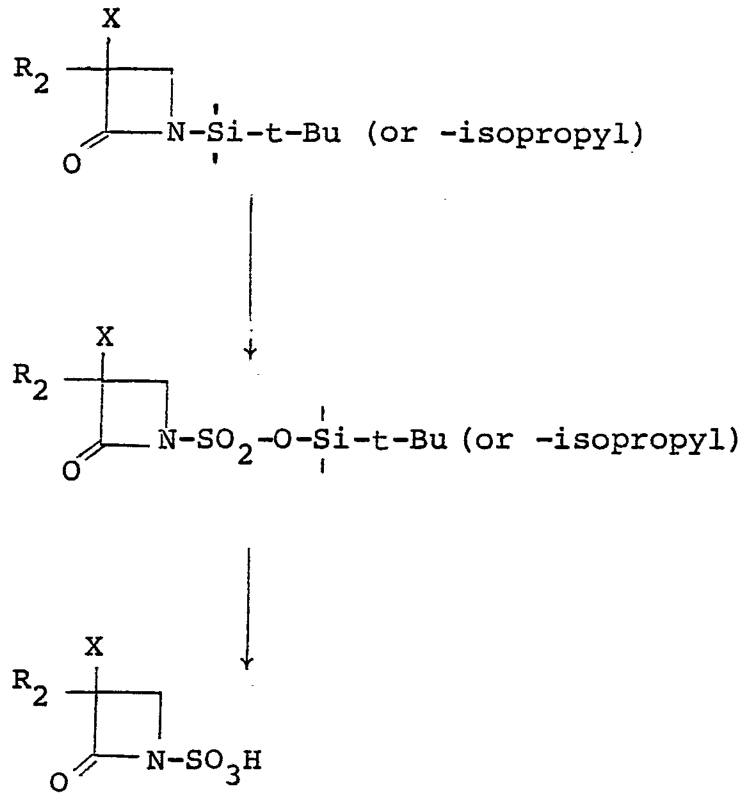

In said formulas, X and $R_2$ are of the same meaning as defined above. "t-Bu" stands for t-butyl.

After the reaction, the compounds (I) can be recovered in any desired purity by subjecting silyl-compound to decomposition or the reaction mixture to a purification/separation procedure known per se, such as solvent extraction, recrystallization and/or chromatography.

The compounds (I) having protective groups are valuable as intermediates for the synthesis of useful medicines and, for example, can be converted to unprotected compounds (I) by elimination of the protective groups.

The elimination of protective groups from azetidine derivatives (I) having protective groups can be effected by selective application of a per se conventional methods such as (1) a method involving the use of an acid, (2) a method involving the use of an iminohalogenating agent and then of an iminoetherifying agent, followed by, upon necessity, hydrolysis, (3) a method involving the use of a base, (4) a method involving the use of thiourea or sodium

N-methyldithiocarbamate, (5) a method involving the use of hydrazine or (6) a method involving reduction.

(1) In the method involving the use of an acid, it employs, depending on the type of protective group and other conditions, such an inorganic acid as hydrochloric acid, sulfuric acid, phosphoric acid, etc., such an organic acid as formic acid, acetic acid, trifluoroacetic acid, propionic acid, benzenesulfonic acid, p-toluenesulfonic acid, etc., acidic ion exchange resins [e.g. Amberlite IR-120 (Rohm and Haas Co., U.S.A.), Amberlite IRC-50 (Rohm and Haas Co., U.S.A.), Dowex 50W (Dow Chemical Co., U.S.A.)], and when necessity requires, a hydrophilic organic solvent (e.g. methanol, ethanol, propanol, acetone, acetonitrile, nitromethane, tetrahydrofuran, dioxane, N,N-dimethylformamide, N,N-dimethylacetamide, dimethylsulfoxide), water or a mixed solvent thereof.

The reaction temperature is about 0 to 30°C, preferably about 0 to 20°C.

(2) As the iminohalogenating agent to be used in the method of eliminating protective groups by using an iminohalogenating agent and then an iminoetherifying agent followed by, when necessary, hydrolysis, there may be used, for example, phosphorus trichloride, phosphorus pentachloride, phosphorus tribromide, phosphorus pentabromide, phosphorus oxychloride, thionyl chloride, phosgene, etc. The reaction temperature is not critical, but the reaction is mostly carried out under cooling (about -20°C) or at room temperature (about 20°C).

As an iminoetherifying agent to be reacted with thus-obtained reaction product, alcohols or metal alkoxides are used. The alcohols include alkanols such as methanol, ethanol, propanol, isopropanol, n-butanol, tert-butanol, etc. as well as those compounds in which the alkyl moieties of the above-mentioned alcohols are substituted with an alkoxy group such as methoxy, ethoxy, propoxy, isopropoxy, butoxy or the like. The metal alkoxides include alkali

metal alkoxides such as sodium alkoxides, potassium alkoxides, etc. and alkaline earth metal alkoxides such as calcium alkoxides, barium alkoxides, etc. derived from such alcohols as above.

The reaction temperature is not critical, but the reaction is mostly carried out at about -50 to 30°C, preferably -20 to 20°C.

These methods, i.e. (3) the method involving the use of a base, (4) the method involving the use of thiourea or sodium N-methyldithiocarbamate, (5) the method invovling the use of hydrazine and (6) the method involving reduction, are carried out by employing the corresponding methods mentioned in the method of removing the protective group of the amino group of 1-t-butyl or isopropylsilyl derivative of the compound (II).

The compounds (I) from which the protective groups are removed can be led to desired salts such as addition salts with an inorganic acid exemplified by hydrochloric acid, sulfuric acid and phosphoric acid, or an organic acid exemplified by acetic acid, p-toluenesulfonic acid, mandelic acid, citric acid and tartaric acid.

Having a sulfo group, the compounds (I) can in general form salts with bases. Therefore, the compounds (I) may be recovered in the form of salts, and the salts recovered may be converted to the free form or to other salts. Furthermore, the compounds (I) obtained in the free form may be converted to salts. The above-mentioned bases are, for example, inorganic bases, such as lithium, potassium, sodium, calcium and ammonia, and organic bases, such as pyridine, colidine, triethylamine and triethanolamine.

When the compounds (I) have free amino groups or carboxyl groups, they can respectively be recovered in the form of addition salts with such inorganic acids as hydrochloric acid, sulfuric acid or phosphoric acid and such organic acids as formic acid, acetic acid or p-toluenesulfonic acid, or in the form of salts with the above-mentioned bases.

According to the circumstances, the compounds (I) may involve stereoisomers (e.g. D-isomer, L-isomer).

These individual isomers as well as mixture thereof can be used as medicines. When a mixture of such isomers is obtained,each isomer can be isolated as necessary by a conventional method of optical resolution.

The compounds (I) are all in a stable powdery form, which are substantially tasteless, odorless and low toxicity, thus being useful as medicines. For example, they have antibacterial activity against certain kinds of Gram-positive bacteria (e.g. Staphylococcus aureus) and Gram-negative bacteria (e.g. Escherichia coli) and have β-lactamase inhibitory activity against β-lactamase which is produced by a resistant bacteria.

The compounds (I) can be used as therapeutic or prophylactic agents for bacterial infections in humans or domestic animals (e.g. mouse, rat, etc.), singly or in combination with β-lactam antibiotics. The compounds (I) can be prepared into, for example, injections, dry-syrups, granules, tablets or capsules, by per se conventional manner, but, preferably, they are used as injections in the form of their salts or hydrates. The daily dose is, for example, generally about 50-1000 mg for an adult human, usually 100-750 mg per day in 2-4 divided doses.

Among the starting compound (II), those in which X stands for hydrogen can be prepared by a similar manner to those described in British Patent No. 1,519,495.

The starting compound (II), including the cases where X stands for hydrogen and methoxy, can be prepared by, for example, subjecting a compound represented by the formula (III):

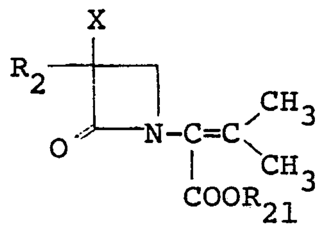

(III)

wherein X and $R_2$ have the same meaning as defined above, and $R_{21}$ stands for ester residue, to oxidation with for example, ozone, alkali metal permanganate (e.g. potassium permanganate, sodium permanganate), alkaline earth metal permanganate (e.g. barium permanganate), osmium tetroxide, lead tetracetate, etc. in tetrahydrofuran, dioxane, N,N-dimethylformamide, N,N-dimethylacetamide, benzene, acetone, pyridine, methanol, ethanol, propanol, butanol, water, chloroform, dichloromethane, carbon tetrachloride or an optional mixture of them.

The ester residue $R_{21}$ of the formula (III) includes, for example, methyl, ethyl, propyl, isopropyl, n-butyl, t-butyl, pentyl, cyclopentyl, cyclohexyl, benzyl, p-nitrobenzyl, benzhydryl, alkoxyalkyl, alkanoyloxymethyl, alkenyl, trichloroethyl, methylsulfonylethyl, benzoylmethyl, methoxybenzyl, trityl, methylthiomethyl, pivaloyloxymethyl, α-acetoxybutyl, etc.

Among the compounds (III), those in which X is methoxy can be prepared by allowing the compound (III) in which X is hydrogen to react with methylate of lithium, sodium or potassium in the presence of methanol and with a halogenating agent such as a halogen (e.g. chlorine or bromine), an N-haloimide (e.g. N-chlorosuccinimide or N-bromosuccinimide), a haloamide (e.g. N-chloroacetamide or N-bromoacetamide), an N-halosulfonamide (e.g. N-chlorobenzenesulfonamide or N-chloro-p-toluenesulfoamide), a 1-halobenzotriazole, or an organic hypochloride (e.g. t-butylhypochloride), in, for example, tetrahydrofuran, dioxane, methylene chloride, chloroform, acetonitrile, methanol, N,N-dimethylformamide, N,N-dimethylacetamide or an optional mixture of them.

More precisely, the starting compound (IV) is produced

by, for example, the following procedures.

Procedure 1)

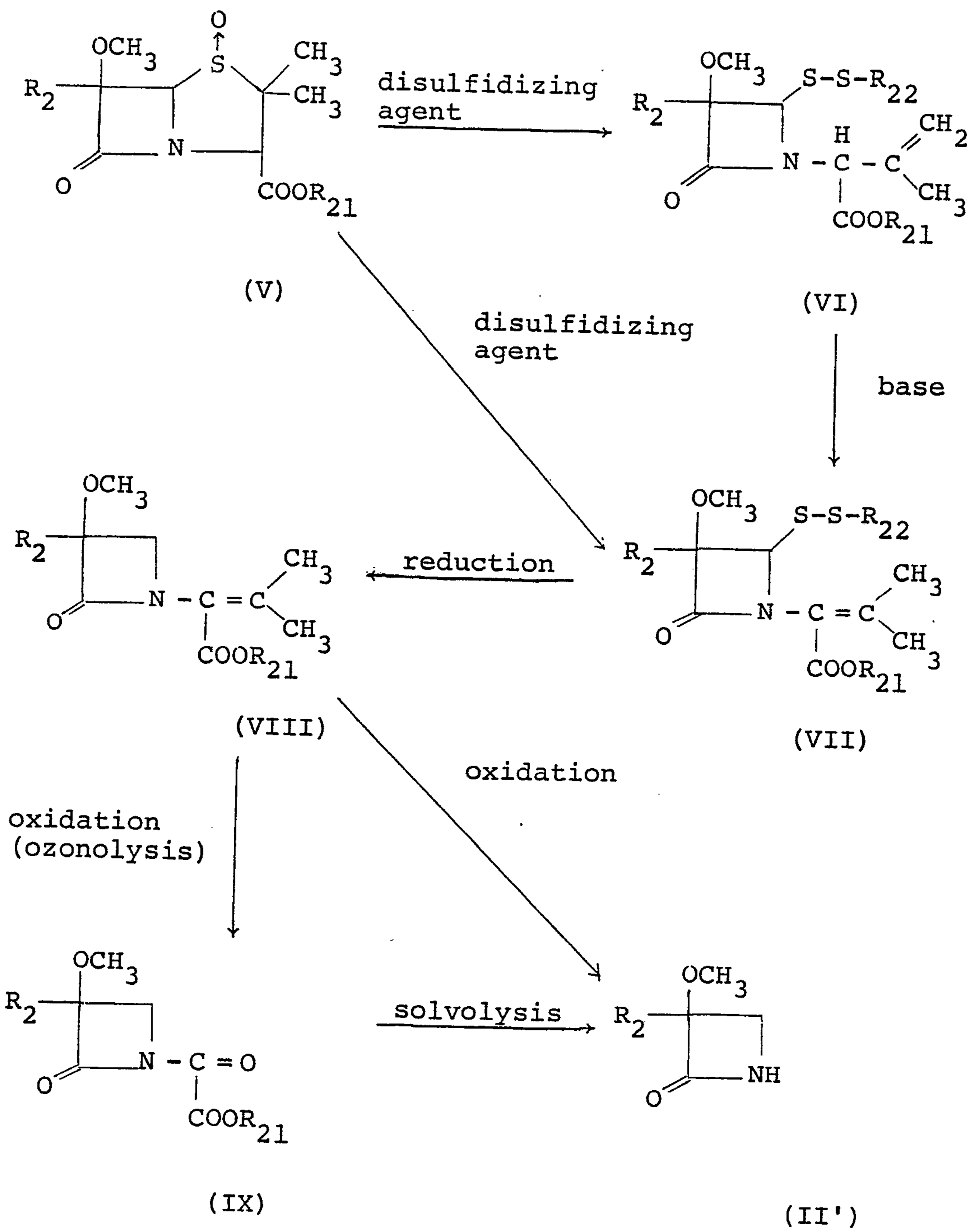


(V) (VI) (VII) (VIII) (IX) (II')

Procedure 2)

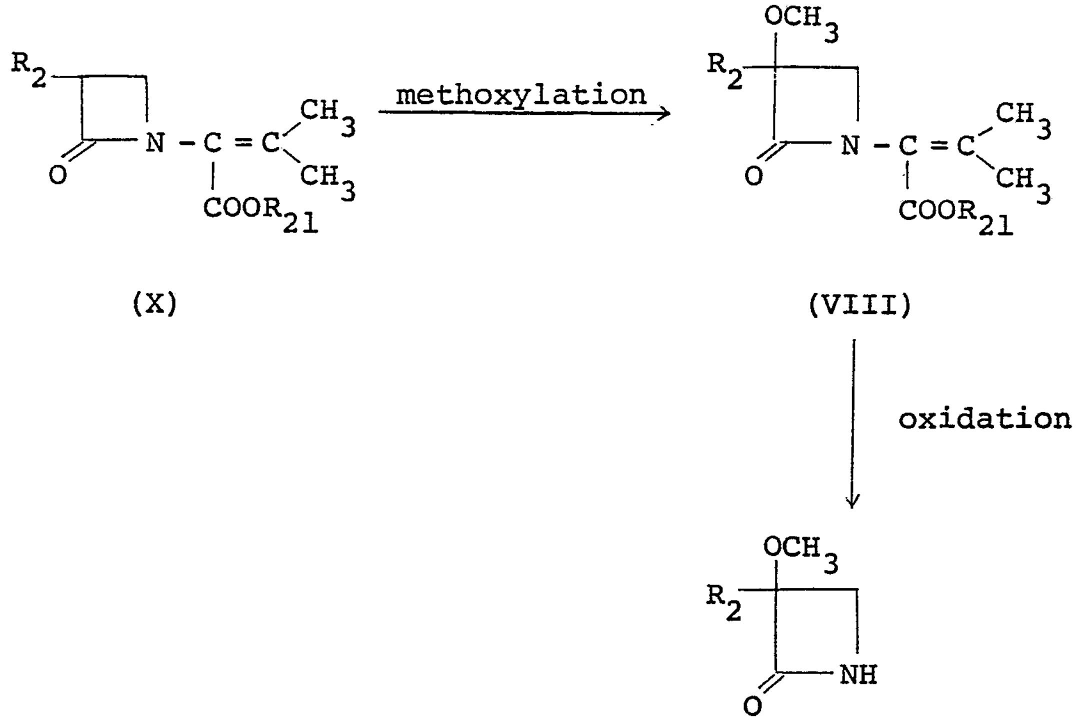


(X) (VIII)

(II')

Procedure 3)

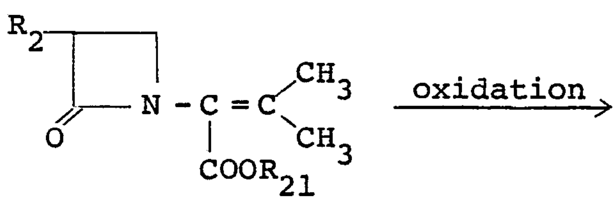


(X) (II")

Regarding the symbols used in the above reaction formula, $R_2$ has the same meaning as defined hereinbefore; $R_{21}$ is an ester residue and $R_{22}$ is a thiol residue.

Exemplary species of the members defined in the above definitions are as follows.

The ester residue $R_{21}$ is of the same groups as mentioned above.

The thiol residue $R_{22}$ include, for example, alkyl groups (e.g. methyl, ethyl, propyl, isopropyl, n-butyl,

t-butyl, isobutyl, n-amyl, vinyl, 1-isopropenyl, etc.), substituted alkyl groups (e.g. methoxymethyl, ethoxymethyl, benzyl, phenethyl, xylylmethyl, p-chlorobenzyl, p-nitrobenzyl, p-methoxybenzyl, etc.), unsubstituted and substitued aryl groups (e.g. phenyl, xylyl, tolyl, naphthyl, chlorophenyl, nitrophenyl, methoxyphenyl, etc.), heterocyclic groups (e.g. benzothiazolyl, benzoxazolyl, thiazolyl, thiadiazolyl, oxazolyl, thienyl, pyridyl, oxadiazolyl, oxatriazolyl, imidazolyl, benzimidazolyl, triazolyl, tetrazolyl, etc.), acyl groups (e.g. acetyl, propionyl, benzoyl, thioacetyl, thiopropionyl, thiobenzoyl, etc.), carbamoyl groups (e.g. methylcarbamoyl, dimethylcarbamoyl, phenylcarbamoyl, etc.), the corresponding and other thiocarbamoyl groups, and groups of the formula:

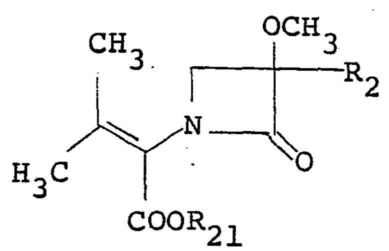

The above-mentioned procedures for producing the azetidine derivative (II) will be described in detail.

Proceudre 1)

This method is related to a fundamental synthetic method for optically active azetidine derivatives (II').

The compound (V) used as a starting material can be easily prepared, for example by the method described in Journal of the American Chemical Society 95, 2401 (1973) or a method analogous thereto. In the first stage, compound (V) is reacted with a disulfidizing agent. The term "disulfidizing agent" is used herein to include all the reactants that are capable of disulfidizing the sulfur in 1-position of compound (V) and, in particular, thiol compounds of the formula $R_5$-SH and disulfides of the

formula $R_{22}$-S-S-$R_{22}$ ($R_{22}$ has the same meaning as defined hereinbefore).

This reaction is carried out the absence of a solvent or in an appropriate solvent. The solvent includes, for example, dioxane, N,N-dimethylacetamide, N,N-dimethylformamide, benzene, toluene, tert-butanol, isopropanol, methyl ethyl ketone, etc., mixtures of such solvents, and other solvents which will not interfere with the reaction. While the reaction temperature is not particularly critical, it is normally advantageous to carry out the reaction at a temperature between 70°C and 150°C.

When a disulfide compound of $R_{22}$-S-S-$R_{22}$ is employed as said disulfidizing agent, the reaction is catalytically accelerated by the presence of an acid or a base. This acid includes, for example, sulfuric acid, phosphoric acid, hydrochloric acid and other mineral acids, p-toluenesulfonic acid, methanesulfonic acid, phenylphosphonic acid, acetic acid, formic acid and other organic acids, and Lewis acids such as ferric chloride, zinc chloride, boron trifluoride, etc. When such an acid is employed, there is predominantly obtained a 1-(α-isopropenyl)azetidine (VI) which contains a double bond in exo-position. The base mentioned above includes, for example, pyridine, quinoline, N,N-dimethylaniline, triethylamine, etc. In this case, depending on the reaction solvent, time, temperature, etc., there is obtained a 1-(α-isopropylidene)azetidine (VII) having a double bond in endo-position in addition to the 1-(2-isopropenyl)azetidine (VI). This 1-(α-isopropylidene)-azetidine (VII) can also be easily obtained by treating 1-(2-isopropenyl)azetidine (VI) with a base. The reaction according to this procedure is preferably carried out in streams of an inert gas such as nitrogen, helium or the like. The useful molar ratio of disulfidizing agent to starting compound (V) depends on the S-nucleophilicity of the disulfidizing agent used but, generally speaking, about 1 to about 10 equivalents of said agent are employed. After

completion of the reaction, the product compound (VI) can be isolated in optional purity by the purification procedures known *per se*, e.g. extraction with solvents, recrystallization, chromatography, etc.

The compound (VI), on treatment with a base, yields the compound (VII). The base used for this purpose may be the above-mentioned base which can be used as a catalyst in the reaction between compound (VI) and disulfidizing agent. In carrying out this reaction, the base need not be employed in a large amount. Thus, relative to compound (VI), about 0.01 to about 0,2 mol equivalent is sufficient. The reaction is generally carried out in a solvent such as dichloromethane, chloroform, benzene, toluene, tert-butanol, methanol, ethanol, tetrahydrofuran, dioxane, methyl ethyl ketone, N,N-dimethylacetamide, N,N-dimethylformamide, etc. or a mixture of such solvents. Any other solvent that will not interfere with the reaction may also be employed. While the reaction temperature is not particularly critical, the reaction proceeds at room temperature in many instances. The product derivative (VII) in which $R_{22}$ is a group of the formula:

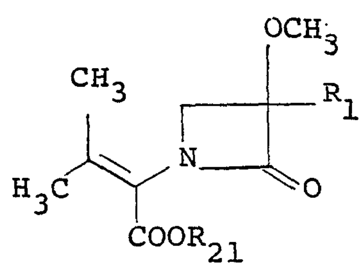

is a compound which is obtained simultaneously or partially in this reaction step and can be converted to compound (VIII) by treatment with a reducing agent.

Then, compound (VII) is subjected to a reductive desulfurization reaction. The reductive desulfurizing agent used for this purpose may, for example, by Raney nickel, Raney cobalt or the like. This reaction is usually carried out in a solvent. The solvent includes, for example,

methanol, ethanol, propanol, tetrahydrofuran, dioxane, ethyl acetate, water, etc., although other common organic solvents which do not interfere with the reaction may also be employed. This reaction proceeds readily under mild conditions, e.g. at room temperature to about 80°C.

The compound (VIII) is then oxidized in order to remove the N-side chain. This oxidation reaction includes an oxidization reaction with an oxidizing agent and a subsequent solvolysis with a solvent or a basic or acidic catalyst.

The oxidizing agent used in the above oxidation reaction includes, for example, ozone, alkali metal permanganate (e.g. potassium permanganate, sodium permanganate, etc.), alkaline earth metal permanganate (e.g. barium permanganate, etc.), osmium tetraoxide, lead tetraacetate, etc. This oxidation reaction is usually carried out in a solvent. This solvent includes, for example, tetrahydrofuran, dioxane, N,N-dimethylformamide, N,N-dimethylacetamide, benzene, acetone, pyridine, methanol, ethanol, propanol, butanol, water, chloroform, dichloromethane, carbon tetrachloride, etc. It may be a mixture of such solvents. The proportion of the oxidizing agent relative to compound (VIII) may be about 1.0 to 4.0 molar equivalents, preferably about 1.0 to 1.2 molar equivalents, although excess ozone if it is used may be employed. While the reaction temperature is not particularly critical, the reaction usually proceeds under cooling or at room temperature. The reaction normally goes to completion with a short time. When a permanganate, for instance, is employed as the oxidizing agent, it is preferable to employ in a buffer solution such as phosphate buffer and carry out the reaction in the neutral pH region so as to minimize the decomposition of starting compound (VI) or/and product compound (I). When ozone is used as said oxidizing agent, the conversion of compound (VIII) to compound (IX) can be effected by ozonolysis e.g. by carrying out the reaction in a solvent

such as chloroform, dichloromethane or carbon tetrachloride, followed by removing the excess ozone and decomposing the ozonide of compound (VIII) with dimethyl sulfide.

The conversion of compound (IX) to compound (II') is effected by subjecting compound (IX) to solvolysis. This reaction is carried out in a suitable solvent and may be optionally conducted with the aid of a basic or acidic catalyst. The base used in such a procedure includes, for instance, inorganic bases such as the hydroxides, carbonates, etc. of alkali metals such as lithium, potassium sodium, etc. or alkaline earth metals such as calcium, magnesium, etc.; organic bases such as metal alkoxides, organic amines,quarternary ammonium salts, etc.; basic ion exchange resins and so forth. The acid used in a similar manner includes inorganic acids and their salts such as hydrochloric acid, sulfuric acid, phophoric acid, zinc chloride, zinc sulfate, ferric chloride, ferric sulfate, etc., organic acids such as formic acid, acetic acid, p-toluenesulfonic acid, trifluoroacetic acid, etc., silica gel, acidic ion exchange resins and so forth. The solvent used for this reaction includes, for example, water, methanol, ethanol, propanol, tetrahydrofuran, dioxane, ethyl acetate, etc. as well as mixtures thereof. Any other solvent that will not interfere with the reaction may also be employed likewise. This reaction usually proceeds easily under mild conditions, e.g. under cooling to a slightly elevated temperature.

The reaction product in each step can be separated in optional purity by purification procedure konwn per se, e.g. extraction with solvents, recrystallization, chromatography, etc.

Procedre 2)

This procedure relates to a fundamental route of synthesis for the production of optically inactive azetidine derivative (II').

The starting compound (X) can be easily prepared by the method described in Molecular Modification in Drug

Design 45, 15 (1964) or a method analogous thereto.

The methoxylation reaction of compound (X) to compound (VIII) is carried out by reacting an alkali metal salt of methanol, which is of the formula $MOCH_3$ (wherein M is an alkali metal), and a halogenating agent with the compound (X) in the presence of methanol. As examples of the alkali metal salt of methanol may be mentioned lithium methoxide, sodium methoxide, potassium methoxide, etc. The halogenating agent is a halogen compound capable of acting as a positive-halogen donor, e.g. halogen (chlorine, bromine, etc.), N-haloimides (N-chlorosuccinimide, N-bromosuccinimide, etc.), haloamides (N-chloroacetamide, N-bromoacetamide, etc.), N-halosulfonamides (N-chlorobenzenesulfonamide, N-chloro-p-toluenesulfonamide, etc.), 1-halobenzotriazoles, organic hypochlorite, etc.). This reaction is carried out in a solvent. Examples of the solvent include tetrahydrofuran, dioxane, dichloromethane, chloroform, acetonitrile, methanol, N,N-dimethylformamide, N,N-dimethylacetamide, etc. as well as various mixtures thereof. Any other solvent that will not interfere with the contemplated reaction may likewise be employed. To carry out the reaction, the starting compound (X) is dissolved or suspended in the above-mentioned solvent and, then, the alkali metal salt of methanol, methanol and halogenating agent are added. The desirable proportions of these agents, relative to each mol of starting compound (X), are not less than 1 mol of methanol, about 1 to 3.5 moles of the alkali metal salt of methanol and about 1 to 2 mols of halogenating agent. The reaction proceeds readily under cooling or at room temperature to about 30°C. The reaction can be quenched by making the reaction system acidic. The suitable acid to quench the reaction may for example be formic acid, acetic acid or trichloroacetic acid. After the reaction has thus been quenched, any excess halogenating agent can be removed by treatment with a reducing agent such as sodium thiosulfate or a trialkyl

phosphite, for instance.

After completion of the above reaction, the product compound (VIII) can be isolated in an optional purity by conventional separatoin-purification procedures, for example by extraction with a solvent, recrystallization, chromatography, etc.

The compound (VIII) is then subjected to procedures similar to the oxidation procedures described hereinbefore in connection with the conversion of compound (VIII) to compound (II'), whereby an optically inactive form of compound (II') is obtained.

Proceudre 3)

In this procedure, the compound (X) obtained for example by the method described in Molecular Modification in Drug Design 45, 15 (1964) or a method analogous thereto is oxidized to compound (II").

This oxidation reaction can be effected by procedures similar to those used in the conversion of compound (VIII) to compound (II") according to the above procedure 1).

The present method of preparing 1-sulfo-2-oxoazetidine derivatives is of industrial advantage in that (1) the intermediate, i.e. a 1-t-butyl or isopropylsilyl derivative of the compound (II), can be recovered in a stable state, and it can be used advantageously as a starting material in the next reaction step, (2) the protective group or the acyl group of the intermediate, i.e. 1-t-butyl or isopropylsilyl derivative of the compound (II), can be easily replaced by some other kind of acyl group, (3) the desired compounds, i.e. 1-sulfo-2-oxoazetidine derivatives, can be produced in a remarkably high yield, because the sulfonation is easily carried out by employing a highly purified starting material, i.e. a 1-t-butyl or isopropylsilyl derivative of the compound (II), (4) even when the starting material, i.e. compound (II), has methoxy group at 3-position, the present method can introduce sulfo group to 1-position of azetidine compound without causing adverse

effect on the methoxy group.

The following reference examples, synthesis examples and examples are given to illustrate this invention in further detail. The symbols used throughout those examples are of the following significances.

| | | |
|---|---|---|
| DMF | : | dimethylformamide |
| DMSO | : | dimethylsulfoxide |
| s | : | singlet |
| d | : | doublet |
| t | : | triplet |
| q | : | quartet |
| m | : | multiplet |
| d.d | : | double doublet |
| ddd | : | sextet |
| quintet | : | quintet |
| broad | : | broad |
| ar. | : | aromatic |
| Me | : | methyl |
| i-Pro | : | isopropyl |
| t-Bu | : | tertiary butyl |
| dec. | : | decomposition |
| c. | : | concentration |

Reference Example 1

(1) A mixture of 4.1 g of methyl 6β-benzyloxycarboxamido-6-methoxypenicillanate-1-oxide and 10 ml of n-amylmercaptan was stirred at 110°C for 24 hours. The excess of n-amylmercaptan was distilled off and the residue was chromatographed on a column of silica gel [eluted with n-hexane-ethyl acetate (2:1)] to give 2.5 g of methyl 4β-n-amyldithio-3β-benzyloxycarboxamido-3α-methoxy-2-oxoazetidine-1-(2-isopropenyl)acetate.

IR $\nu_{max}^{KBr}cm^{-1}$: 3300, 1767, 1736

NMR($CDCl_3$, ppm): 0.93(t,$CH_3$), 1.2-1.7(m,$-CH_2-$), 1.92(s,$CH_3$), 2.76(t,$-S-CH_2-$), 3.60(s,$CH_3$), 3.83(s,$CH_3$). 4.92(s,-CH-), 5.07(s, -CH-), 5.20(m,$=CH_2$), 5.23(s,$-CH_2-$), 5.66(s,NH), 7.42(s,ar.H).

(2) 0.15 g of triethylamine was added to a solution of 2.3 g of methyl 4β-n-amyldithio-3β-benzyloxycarboxamido-3α-methoxy-2-oxoazetidine-1-(2-isopropenyl)acetate in 60 ml of methylene chloride, and the mixture was stirred at room temperature for 1.5 hours. The solvent was distilled off and the residue was chromatographed on a column of silica gel [eluted with n-hexane-ethyl acetate (4:1)] to give 2.2 g of methyl 4β-n-amyldithio-β-benzyloxycarboxamido-3α-methoxy-2-oxoazetidine-1-(α-isopropylidene)acetate.

IR $\nu_{max}^{KBr}cm^{-1}$: 3300, 1768, 1735.

NMR($CDCl_3$, ppm): 0.92(t,$CH_3$), 1.15-1.98(m,$-CH_2-$), 2.08 (s,$CH_3$), 2.32(s,$CH_3$), 2.65(t,$-S-CH_2-$), 3.64(s,$CH_3$), 3.83(s,$CH_3$), 5.23(s,$-CH_2-$), 5.32(s,-CH-), 5.70(s,NH), 7.42(s,ar.H).

(3) 18 ml of Raney-nickel was added to a solution of 2.1 g of methyl 4β-n-amyldithio-3β-benzyloxycarboxamido-3α-methoxy-2-oxoazetidine-1-(α-isopropylidene)acetate in 40 ml of ethanol, followed by stirring at room temperature for one hour. The Raney-nickel was filtered off, and the solvent in the filtrate was evaporated. The residue was chromatographed on a column of silica gel [eluted with n-hexane-ethyl acetate (3:1)] to give 0.62 g of methyl 3-benzyloxycarboxamido-3-methoxy-2-oxoazetidine-1-(α-isopropylidene)acetate.

IR $\nu_{max}^{KBr}cm^{-1}$: 1760, 1718, 1510.

NMR($CDCl_3$, ppm): 1.93(s,$CH_3$), 2.20(s,$CH_3$), 3.50(s,$CH_3$), 3.70(s,$CH_3$), 3.91(dd,J=4.6Hz,$C_4$-H), 5.13(s,$-CH_2-$), 6.03(s,NH), 7.26(s,ar.H).

(4) 6.0 g of methyl 3-benzyloxycarboxamido-3-methoxy-2-oxoazetidin-1-(α-isopropylidene)acetate was dissolved in 150 ml of methylene chloride, and ozone gas was introduced into the solution at -50°C to -30°C. The reaction mixture became blue after one hour. Then, the excess ozone was removed by the introduction of nitrogen gas, followed by addition of dimethyl sulfide. After stirring at room temperature for an hour, the reaction mixture was washed

with water and the solvent was evaporated to give 6.1 g of methyl 3-benzyloxycarboxamido-3-methoxy-2-oxoazetidine-1-α-ketoacetate. This product was dissolved in 75 ml of methanol, and to the solution was added 19 ml of 0.002% sodium methoxide in methanol. After stirring at room temperature for 15 minutes, 0.3 g of acetic acid was added, and the solvent was distilled off. The residue was dissolved in ethyl acetate and the solution was washed with water. After removal of the solvent, the residue was chromatographed on a column of silica gel [eluted with ethyl acetate-n-hexane (1:1)] to give 2.7 g of 3-benzyloxycarboxamido-3-methoxy-2-oxoazetidine as crystals.

Optical rotation: $[\alpha]_D^{25°}$ + 68.2° (c=1, MeOH).

IR $\nu_{max}^{CHCl_3}cm^{-1}$: 3420, 1774, 1723.

NMR($CDCl_3$, ppm): 3.45(s,$CH_3$), 3.60(d,J=6Hz,$C_4$-H), 3.80 (d,J=6Hz,$C_4$-H), 5.14(s,-$CH_2$-), 6.74(broad s,NH), 7.34 (s,ar.H).

(5) 5.7 ml of tert-butyl hypochlorite was added to a solution of 14 g of methyl 3-benzyloxycarboxamido-2-oxoazetidine-1-(α-isopropylidene)acetate in 400 ml of dry tetrahydrofuran and, then, a solution of 0.348 g lithium in 32 ml methanol was added with stirring at -30 to -20°C. The mixture was maintained at -15°C for 30 minutes, and 1 ml of acetic acid was added, and the solvent was distilled off. The residue was dissolved in ethyl acetate, and after washing with water, the solvent was distilled off. The residue was chromatographed on a column of silica gel [eluted with n-hexane-ethyl acetate (1:1)] to give 11.1 g of methyl 3-benzyloxycarboxamido-3-methoxy-2-oxoazetidine-1-(α-isopropylidene)acetate as crystals.

m.p.: 77°C

IR $\nu_{max}^{KBr}cm^{-1}$: 1761, 1723.

NMR($CDCl_3$, ppm): 1.91(s,$CH_3$), 2.22(s,$CH_3$), 3.53(s,$CH_3$), 3.73(s,$CH_3$), 4.1(ABq,J=6Hz,$C_4$-H), 5.20(s,-$CH_2$-), 6.58(s,NH), 7.36(s,ar.H).

(6) 7.2 g of methyl 3-benzyloxycarboxamido-3-methoxy-2-

oxoazetidine-1-(α-isopropylidene)acetate was dissolved in 150 ml of methylene chloride and ozone gas was introduced into the solution at -50°C to -30°C. The reaction mixture became blue after 55 minutes. Then, nitrogen gas was introduced until the solution became colorless. Then, 6 ml of dimethyl sulphide was added, followed by stirring at room temperature for 30 minutes. The reaction mixture was washed with water and the solvent was evaporated to give 8.1 g of methyl 3-benzyloxycarboxamido-3-methoxy-2-oxoazetidine-1-α-ketoacetate. This product was dissolved in 100 ml of methanol, followed by the addition of 25 ml of 0.002% sodium methoxide in methanol. After stirring at room temperature for 15 minutes, the solvent was distilled off, and the residue was dissolved in ethyl acetate. The solution was washed with water, and the solvent was evaporated to give 3.3 g of 3-benzyloxycarboxamido-3-methoxy-2-oxoazetidine as crystals. In IR and NMR, this product was identical with the optically active compound obtained in Reference Example 1-(4).

Optical rotation: $[\alpha]_D^{25°}$ 0° (c=1, MeOH).

Reference Example 2

(1) A solution of 47.5 g of methyl 3-phenylacetamido-2-oxoazetidine-1-(α-isopropylidene)acetate in 750 ml of methylene chloride was cooled to a temperature below -70°C, followed by the addition of 93.7 g of finely divided phosphorus pentachloride and 71.2 g of pyridine. The mixture was stirred under ice-water-cooling for 70 minutes. The reaction mixture was cooled to -70°C and after addition of 150 ml of n-butanol, the temperature was raised gradually to 0°C. After an hour, 300 ml of ice-water was added and the water layer was adjusted to pH 6.2 with sodium hydrogen carbonate. It was extracted with chloroform and the solvent was distilled off. By the above procedure 26 g of methyl 3-amino-2-oxoazetidine-1-(α-isopropylidene)acetate was obtained.

IR $\nu_{max}^{CHCl_3}cm^{-1}$: 3400, 3330, 1750, 1720.

NMR($CDCl_3$, ppm): 1.90(s,$CH_3$), 2.04(br.s.$NH_2$), 2.16(s, $CH_3$), 3.2-3.9(m,$-CH_2-$), 3.73(s,$CH_3$), 4.28(m,-CH-).

(2) While a solution of 58 g of methyl 3-amino-2-oxoazetidine-1-(α-isopropylidene)acetate in 240 ml of methylene chloride was stirred under ice-cooling, 120 ml of propylene oxide and, then, 56.3 g of carbobenzoxy chloride were added. The temperature of the reaction mixture was raised to room temperature and, then, stirred for 30 minutes. The solvent was distilled off and diethyl ether was added to the residue, whereupon crystals separate out. By the above procedure 82.6 g of methyl 3-benzyloxycarboxamido-2-oxoazetidine-1-(α-isopropylidene)acetate was obtained.

IR $\nu_{max}^{KBr}cm^{-1}$: 3280, 1738, 1710.

NMR($CDCl_3$, ppm): 1.95(s,$CH_3$), 2.19(s,$CH_3$), 3.4-3.9(m, $-CH_2-$), 3.74(s,$OCH_3$), 4.89(m,-CH-), 5.11(s,$-CH_2-$), 5.66(d,NH), 7.34(s,ar.H).

(3) 13.3 g of methyl 3-benzyloxycarboxamido-2-oxoazetidine-1-(α-isopropylidene)acetate was dissolved in 400 ml of methylene chloride, and ozone gas was introduced under cooling at -30°C to -20°C. The reaction mixture became blue after 2 hours. Nitrogen gas was introduced to remove the excess ozone and, after addition of 20 ml of dimethyl sulphide, the mixture was stirred at room temperature for an hour. The reaction mixture was washed with water and the solvent was distilled off to give 19.9 g of methyl 3-benzyloxycarboxamido-2-oxoazetidine-1-α-ketoacetate. This product was dissolved in 200 ml of methanol, then 30 ml of 0.002% sodium methoxide in methanol were added, and the mixture was stirred at room temperature for 15 minutes. To this reaction mixture was added 0.5 g of acetic acid, the solvent was distilled off and cold water-methanol (3:1) was added, whereby 7.62 g of 3-benzyloxycarboxamido-2-oxoazetidine was obtained.

IR $\nu_{max}^{KBr}cm^{-1}$: 3350, 1740, 1725, 1700.

NMR(DMSO-$d_6$, ppm): 3.08-3.40(m,$-CH_2-$), 4.63(m,-CH), 5.00

(s,$-CH_2-$), 7.28(s,ar.H), 7.80(s,NH), 7.85(d,NH).

Reference Example 3

To a solution of 180 mg of 1-tert-butyldimethylsilyl-3-phenylacetamido-2-oxoazetidine in 2 ml of dimethylformamide, was added catalytic amount of tetra-n-butylammonium fluoride, followed by stirring at room temperature for 25 minutes, then dimethylformamide was distilled off under reduced pressure. To the residue, was added a solution of ethyl acetate-chloroform-methanol (3:3:1) and the resulting precipitate was collected by filtration to give 97.2 mg of 3-phenylacetamido-2-oxoazetidine.

Reference Example 4

A mixture of 2.6 g of 1-tert-butyldimethylsilyl-3-phenylacetamido-3-methoxy-2-oxoazetidine, 0.4 g of potassium fluoride and 30 ml of methanol was stirred at 20°C for 15 minutes. The resultant was evaporated under reduced pressure, and the residue was chromatographed on a column of silica gel [eluted with ethyl acetate-n-hexane (2:1)] to give 1.4 g of 3-phenylacetamido-3-methoxy-2-oxoazetidine.

IR $\nu_{max}^{KBr}cm^{-1}$: 1750, 1695, 1525, 1260.

NMR($CDCl_3$, ppm): 3.35(s,$CH_3$), 3.60(s,$-CH_2-$), 3.56, 3.79 (each d,J=6Hz,$C_4$-H), 6.68(s,NH), 7.20(s,ar.H), 7.50 (s,NH).

Example 1

1.89 g of tert-butyldimethylsilyl chloride was added to a solution of 2.2 g of 3-benzyloxycarboxamido-2-oxoazetidine obtained in Reference Example 2 and 1.52 g of triethylamine in 11 ml of dimethylformamide under ice-cooling, followed by stirring at room temperature for an hour. The reaction mixture was poured onto ice-water, extracted with ethyl acetate. The extract was dried over anhydrous magnesium sulfate and, concentrated to give 3.2 g of 1-tert-butyldimethylsilyl-3-benzyloxycarboxamido-2-oxoazetidine.

IR $\nu_{max}^{KBr}cm^{-1}$: 1740, 1720, 1520, 1250.

NMR($CDCl_3$, ppm): 0.20(s,$SiMe_2$), 0.93(s,$CH_3$), 3.26(dd,

J=3.6Hz,$C_4$-βH), 3.53(t,J=6Hz, $C_4$-αH), 4.93(m,$C_3$-H), 5.16(s,-$CH_2$-), 5.76(d,J=8Hz,NH), 7.36(s,ar.H).

Example 2

To a solution of 2.5 g of 3-benzyloxycarboxamido-3-methoxy-2-oxoazetidine obtained in Reference Example 1 and 1.52 g of triethylamine in 12 ml of dimethylformamide was added 1.89 g of tert-butyldimethylsilyl chloride with stirring under ice-cooling followed by stirring room temperature for 2 hours. The reaction mixture was poured onto ice-water, extracted with ethyl acetate. The extract was, after drying over anhydrous magnesium sulfate, concentrated to give 3.05 g of 1-tert-butyldimethylsilyl-3-benzyloxycarboxamido-3-methoxy-2-oxoazetidine as crystals.

mp 86-87°C (colorless needles)

IR $\nu_{max}^{KBr}cm^{-1}$: 3300, 1750 (shoulder), 1720, 1260.

NMR($CDCl_3$, ppm): 0.20(s,$SiMe_2$), 0.93(s,$CH_3$), 3.50(s,OMe), 3.60(d,J=6Hz,$C_4$-H), 3.80(d,J=6Hz,$C_4$-H), 5.20(s,-$CH_2$-), 6.40(broad s,NH), 7.40(s.ar.H).

Example 3

To a solution of 460 mg of 3-benzyloxycarboxamido-3-methoxy-2-oxoazetidine obtained in Reference Example 1 and 280 mg of triethylamine in 2 ml of dimethylformamide was added, 251 mg of isopropyldimethylsilyl chloride with stirring under ice-cooling, followed by stirring at room temperature for 2 hours. The reaction mixture was filtered and the filtrate was concentrated under reduced pressure. The oily residue was chromatographed on a column of fluorisil [eluted with ethyl acetate-n-hexane (1:1)] to give 484 mg of 1-isopropyldimethylsilyl-3-benzyloxycarboxamido-3-methoxy-2-oxoazetidine as crystals.

mp. 57-59°C (dec.) (colorless plates).

IR $\nu_{max}^{KBr}cm^{-1}$: 3290, 1740, 1720, 1505, 1260.

NMR($CDCl_3$, ppm): 0.23(s,$SiMe_2$), 1.00(s,$CH_3$), 3.46(s,OMe), 3.52, 3.71(each d,J=6Hz,$C_4$-H), 5.13(s,-$CH_2$-), 6.73(broad s,NH), 7.30(s,ar.H).

Example 4

To a suspension of 3.1 g of 3-phenylacetamido-2-

oxoazetidine and 1.69 g of triethylamine in 35 ml of dimethylformamide was added 2.41 g of tert-butyldimethylsylyl chloride under ice-cooling, followed by stirring at room temperature for 3 hours. The reaction mixture was poured onto ice-water-ethyl acetate and ethyl acetate layer was recovered, washed with water, dried over anhydrous magnesium sulfate and concentrated to give 4.77 g of 1-tert-butyldimethylsilyl-3-phenylacetamido-2-oxoazetidine as colorless oil.

IR $\nu_{max}^{film}cm^{-1}$: 3280, 1745, 1730 (shoulder), 1655, 1530, 1205.

NMR($CDCl_3$, ppm): 0.20(s,$SiMe_2$), 0.93(s,t-Bu), 3.20(dd, J=3,6Hz,$C_4$-βH), 5.53(t,J=6Hz,$C_4$-αH), 3.60(s,$-CH_2-$), 4.93(m,$C_3$-H), 6.73(d.J=8Hz,NH), 7.36(s,ar.H).

Example 5

To a solution of 408 mg of 3-phenylacetamido-2-oxoazetidine and 300 mg of triethylamine in 2 ml of dimethylformamide, was added 274 mg of isopropyldimethylsilyl chloride, followed by stirring at room temperature for 30 minutes. The reaction mixture was poured onto ice-water followed by extraction with ethyl acetate. The extract was washed with water, dried over anhydrous magnesium sulfate and concentrated. The resulting oily residue was chromatographed on a silica gel [eluted with ethyl acetate-n-hexane (1:1)], whereby 250 mg of 1-isopropyldimethylsilyl-3-phenylacetamido-2-oxoazetidine was obtained.

IR $\nu_{max}^{film}cm^{-1}$: 3280, 1745 (shoulder), 1725, 1655, 1535, 1205.

NMR($CDCl_3$, ppm): 0.23(s,$SiMe_2$), 1.00(s,$CH_3$), 3.10(dd, J=3.6Hz,$C_4$-βH), 3.45(t,J=6Hz,$C_4$-αH), 3.53(s,$-CH_2-$), 4.93(m,$C_3$-H),7.20(s,ar.H), 7.40(d,J=8Hz,NH).

Example 6

A mixture of 349 mg of 1-tert-butyldimethylsilyl-3-methoxy-3-benzyloxycarboxamido-2-oxoazetidine obtained in Example 2, 50 mg of palladium black and 5 ml of tetrahydrofuran was stirred in a stream of hydrogen for 20

minutes, whereby 230 mg of 3-amino-1-tert-butyldimethylsilyl-3-methoxy-2-oxoazetidine was obtained.

IR $\nu_{max}^{film}cm^{-1}$: 3370, 3300, 1738, 1250, 1195.

NMR($CDCl_3$, ppm): 0.23(s,$SiMe_2$), 0.97(s,t-Bu), 2.18 (broad s,$NH_2$), 3.32, 3.43(each d,J=6Hz,$C_4$-H), 3.43 (s,$CH_3$).

To a solution of 5 ml of tetrahydrofuran containing 230 mg of 1-tert-butyldimethylsilyl-3-amino-3-methoxy-2-oxoazetidine as mentioned above, were added 136 mg of phenylacetic acid and 206 mg of dicyclohexylcarbodiimide under ice-cooling, followed by stirring at the same temperature for 2 hours. The reaction mixture was left standing over night at room temperature. The reaction mixture was filtered and the filtrate was concentrated under reduced pressure. The residue was chromatographed on a column of silica gel [eluted with ethyl acetate-n-hexane (1:2)], whereby 238 mg of 1-tert-butyl-dimethylsilyl-3-phenylacetamido-3-methoxy-2-oxoazetidine was obtained.

IR $\nu_{max}^{KBr}cm^{-1}$: 3280, 1740, 1680, 1520, 1260

NMR($CDCl_3$, ppm): 0.20(s,$SiMe_2$), 0.93(s,$CH_3$), 3.33(s, OMe), ,3.53(d,J=6Hz,$C_4$-H), 3.60(s,-$CH_2$-), 3.76(d, J=6Hz,$C_4$-H), 7.23(s,ar.H), 8.03(broad s,NH).

Example 7

A mixture of 335 mg of 1-tert-butyldimethylsilyl-3-benzyloxycarboxamido-2-oxoazetidine obtained in Reference Example 1, 50 mg of palladium black and 5 ml of tetrahydrofuran was stirred in a stream of hydrogen for 20 minutes, whereby 216 mg of 3-amino-1-tert-butyldimethylsilyl-2-oxoazetidine was obtained.

IR $\nu_{max}^{film}cm^{-1}$: 3350, 1735 (shoulder), 1720, 1200.

NMR($CDCl_3$, ppm): 0.25(s,$SiMe_2$), 0.95(s,$CH_3$), 1.85(broad s,$NH_2$), 2.96(dd,J=3,6Hz,$C_4$-βH), 3.50(t,J=6Hz,$C_4$-αH), 4.23(dd,J=3,6Hz,$C_3$-H).

To a solution of 20 ml of tetrahydrofuran containing 1.2g of 1-tert-butyldimethylsilyl-3-animoazetidine as mentioned above, was added 910 mg of triethylamine, followed by adding

1 g of phenylacetyl chloride with stirring under ice-cooling. After stirring at room temperature for an hour, the reaction mixture was filtered and the filtrate was concentrated under reduced pressure. The oily residue was chromatographed on a column of silica gel [eluted with ethylacetate-n-hexane (1:2)], whereby 1.55 g of 1-tert-butyldimethylsilyl-3-phenylacetamido-2-oxoazetidine was obtained.

IR $\nu_{max}^{KBr}cm^{-1}$: 1740, 1655, 1530, 1200.

NMR($CDCl_3$,ppm): 0.20(s,$SiMe_2$), 0.93(s,$CH_3$), 3.20(dd, J=3,6Hz,$C_4$-βH), 3.53(t,J=6Hz,$C_4$-αH), 3.60(s,$-CH_2-$), 4.93(m,$C_3$-H), 6.73(d,J=8Hz,NH), 7.36(s,ar.H).

Example 8

To a solution of 30 ml of tetrahydrofuran containing 1382 mg of 1-tert butyldimethylsilyl-3-amino-3-methoxy-2-oxoazetidine obtained in Example 6, was added 910 mg of triethylamine, followed by adding 928 mg of phenylacetyl chloride with stirring under cooling at -20°C. After stirring at room temperature for an hour, the reaction mixture was filtered and the filtrate was concentrated under reduced pressure. The residue was chromatographed on a column of silica gel [eluted with ethyl acetate-n-hexane (1:2)] to give 1.8 g of 1-tert-butyldimethylsilyl-3-phenylacetamido-3-methoxy-2-oxoazetidine. IR and NMR of this sample was identical with that obtained in Example 6.

Example 9

To a solution of 10 ml of tetrahydrofuran containing 419 g of 1-isopropyldimethylsilyl-3-amino-3-methoxy-2-oxoazetidine which was prepared in accordance with the procedure of Example 6, from 1-isopropyldimethylsilyl-3-benzyloxycarboxamido-3-methoxy-2-oxoazetidine obtained in Example 3, was added 400 mg of triethylamine, followed by adding 300 mg of phenylacetyl chloride under acetone-dry-ice cooling. In accordance with the procedure of Example 8, there was obtained 250 mg of 1-isopropyldimethylsilyl-3-phenylacetamido-3-methoxy-2-oxoazetidine.

IR $\nu_{max}^{KBr}cm^{-1}$: 3280, 1740, 1665, 1540, 1520, 1240.

BAD ORIGINAL

NMR($CDCl_3$, ppm): 0.23(s,$SiMe_2$), 1.00(s,$CH_3$), 3.36(s, OMe), 3.55, 3.79(each d,J=6Hz,$C_4$-H), 3.63(s,$-CH_2-$), 7.26(s,ar.H), 7.63(broad s,NH).

Example 10

To a solution of 15 ml of tetrahydrofuran containing 691 mg of 1-tert-butyldimethylsilyl-3-amino-3-methoxy-2-oxoazetidine obtained in Example 6, was added 400 mg of triethylamine, followed by adding 30 ml of methylene chloride at -20°C containing acid chloride prepared from 1.2 equivalent of Vilsmeyer reagent and 917 mg of 2-isopropoxyimino-2-(2-chloroacetamidothiazol-4-yl)acetic acid. After stirring at room temperature for an hour, the reaction mixture was filtered and the filtrate was concentrated under reduced pressure. The oily residue was chromatographed on a column of silica gel [eluted with ethyl acetate-n-hexane (1:1)] to give 660 mg of 1-tert-butyldimethylsilyl-3-[2-isopropoxyimino-2-(2-chloroacetamidothiazol-4-yl)acetamido]-3-methoxy-2-oxoazetidine.

IR $\nu_{max}^{KBr}cm^{-1}$: 3400, 3200, 1735, 1690 (shoulder), 1665, 1540, 1255.

NMR($CDCl_3$, ppm): 0.28, 0.30(each s, $Si{<}^{Me}_{Me}$), 1.00(s,$CH_3$), 1.15, 1.20(each d,J=6Hz,$-{<}^{Me}_{Me}$), 3.57(s,oMe), 3.70(d, J=7Hz,$C_4$-H), 3.95(d,J=7Hz,$C_4$-H), 4.25(s,$-CH_2-$), 4.28 (quintet, J=6Hz,$-CH{<}$), 7.40(s, 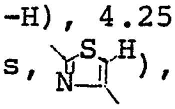), 8.00(broad, s, NH), 10.66(broad s,NH).

Example 11

195 mg of 3-amino-1-tert-butyldimethylsilyl-2-oxoazetidine obtained in Example 7 and 514 mg of 2-tert-butoxycarbonylisopropoxyimino-2-(2-tritylaminothiazol-4-yl)acetic acid were worked up in the manner of Example 5 to give 450 mg of 1-tert-butyldimethylsilyl-3-[2-[1-(tert-butoxycarbonyl)isopropoxyimino]-2-(2-tritylaminothiazol-4-yl)-acetamido]-2-oxoazetidine.

IR $\nu_{max}^{KBr}cm^{-1}$: 1745, 1730, 1675, 1520.

NMR($CDCl_3$, ppm): 0.22, 0.26(each s,$SiMe_2$), 0.97(s,$CH_3$), 1.46(s,$CH_3$), 1.57, 1.60(each s,$-O\underset{Me}{\overset{Me}{C}}CO-$), 3.26(dd,

J=4,6Hz,$C_4$-βH), 3.58(t,J=6Hz,$C_3$-αH), 5.20(ddd,J=4,6,8Hz,$C_3$-H), 6.76(s, 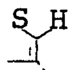), 6.84(broad s,NH), 7.30(s, ar.H), 8.01(d,J=8Hz,,NH).

Example 12

1.0 g of 3-benzyloxycarboxamido-1-tert-butyldimethylsilyl-3-methoxy-2-oxoazetidine and 0.843 g of D-2-(2-oxoimidazolidin-1-yl-carboxamido)-2-thienylacetic acid were worked up in the manner of Example 10 to give 0.197 g and 0.181 g respectively of two diastereoisomers of 1-tert-butyldimethylsilyl-3-methoxy-3-[D-2-(2-oxoimidazolidin-1-yl-carboxamido)-2-thienylacetamido-2-oxoazetidine.

Compound A

IR $\nu_{max}^{KBr}cm^{-1}$: 3260, 2940, 2920, 1750, 1730, 1715, 1688, 1640, 1520, 1255.

NMR($DMSO-d_6$, ppm): 0.22(s,$CH_3$), 0.94(s,t-Bu), 3.19(s,$CH_3$), 3.20-3.44(m,$-CH_2-$), 3.53(dd,J=7,14Hz,$C_4$-H), 3.60-3.86(m,$-CH_2-$), 5.84(d,J=8Hz,-CH-), 6.90-7.16(m, 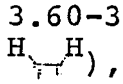), 7.40-7.50(m, 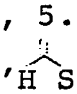), 7.56(s,NH), 9.00(d,J=8Hz, NH), 9.58(s,NH).

Compound B

IR $\nu_{max}^{KBr}cm^{-1}$: 3260, 2940, 2920, 1750, 1720, 1650, 1515, 1250.

NMR($DMSO-d_6$, ppm): 0.15(s,$CH_3$), 0.18(s,$CH_3$), 0.85(s,t-Bu), 3.18-3.44(m,$-CH_2-$), 3.34(s,$CH_3$), 3.46(dd,J=7,9Hz,$C_4$-H), 3.60-3.88(m,$-CH_2-$), 5.82(d,J=8Hz,-CH-), 6.90-7.10(m, 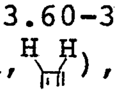), 7.38-7.50(m, 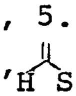), 7.56(s,NH), 8.97(d,J=8Hz,NH), 9.61(s,-NH-).

Example 13

1.09 g of 3-benzyloxycarboxamido-1-tert-butyldimethylsilyl-3-methoxy-2-oxoazetidine and 0.961 g of D-2-(3-ethyl-2-oxoimidazolidin-1-yl-carboxamido)-2-phenylacetic acid were worked up in the manner of Example 10 to give 0.277 g of 1-tert-butyldimethylsilyl-3-[2-(3-ethyl-2-oxoimidazolidin-1-yl-carboxamido)-2-phenylacetamido]-3-methoxy-2-oxoazetidine(diastereoisomer A).

IR $\nu_{max}^{KBr}cm^{-1}$: 3270, 2950, 2930, 1745, 1718, 1675, 1650,

1522, 1480, 1260.

NMR($CDCl_3$, ppm): 0.23(s,$CH_3$), 0.94(s,t-Bu), 1.14(t, J=7Hz,$CH_3$), 3.21(s,$CH_3$), 3.22-3.70(m,$-CH_2-$,$C_4$-H), 5.70-3.96(m,$-CH_2-$), 5.45(d,J=7Hz,-CH-), 7.06-7.50 (m,ar.H,NH), 9.18(d,J=7Hz,NH).

Further, there was obtained 0.287 g (diastereoisomer B).

IR $\nu_{max}^{KBr} cm^{-1}$: 3280, 2950, 2930, 1745, 1718, 1675, 1655, 1520, 1482, 1262.

Example 14

1.0 g of 3-benzyloxycarboxamido-1-tert-butyldimethylsilyl-3-methoxy-2-oxoazetidine and 0.947 g of 2-(2-methanesulfonamidothiazol-4-yl)-2-isopropoxyiminoacetic acid(syn-isomer) were worked up in the manner of Example 10 to give 1.025 g of 1-tert-butyldimethylsilyl-3-methoxy-3-[2-(2-methanesulfonamidothiazol-4-yl)-2-isopropoxyimino-acetamido]-2-oxoazetidine.

Syn-isomer:

IR $\nu_{max}^{KBr} cm^{-1}$: 3240, 2945, 2920, 1730, 1670, 1525, 1248, 1132.

NMR($CDCl_3$, ppm): 0.26(s,$CH_3$), 0.97(s,t-Bu), 1.27(d, J=6Hz,$CH_3$), 3.02(s,$CH_3$), 3.51(s,$CH_3$), 3.68(dd,J=7,11Hz, $C_4$-H), 4.56(m,-CH-), 7.15(s, 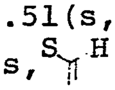 ), 8.18(broad s,NH).

Example 15

0.729 g of 3-benzyloxycarboxamido-1-tert-butyl-dimethylsilyl-3-methoxy-2-oxoazetidine and 0.465 g of 2-azido-2-(3-chlorophenyl)acetic acid were worked up in the manner of Example 10 to give 0.433 g of 3-[2-azido-2-(3-chlorophenyl)acetamido]-1-tertbutyldimethylsilyl-3-methoxy-2-oxoazetidine.

IR $\nu_{max}^{neat} cm^{-1}$: 3260, 2940, 2920, 2100, 1730, 1695, 1248.

NMR($CDCl_3$, ppm): 0.22(s,$CH_3$), 0.84, 0.92(each s,t-Bu), 3.28, 3.38(each s,$CH_3$), 3.57-3.97(m,$C_4$-H), 5.07, 5.09(each s,-CH-), 7.37-7.60(m,ar.H), 8.67(s,NH).

Example 16

1.09 g of 3-benzyloxycarboxamido-1-tert-butyldimethyl-

silyl-3-methoxy-2-oxoazetidine and 0.585 g of 2-azido-2-phenylacetic acid were worked up in the manner of Example 10 to give 0.59 g of 3-(2-azido-2-phenylacetamido)-1-tert-butyldimethylsilyl-3-methoxy-2-oxoazetidine.

IR $\nu_{max}^{neat}cm^{-1}$: 3260, 2940, 2920, 1740, 1682, 1250.

Example 17

To a solution of 0.097 g of dimethylformamide in 5 ml of methylene chloride was added 0.08 ml of diphosgene at -10°C, and the mixture was stirred for 30 minutes at a room temperature. After cooling at -70°C, to the mixture was added dropwise a solution of 0.494 g of 2-(2-chloroacetamido-thiazol-4-yl)-2-(2-trimethylsilylethoxyimino)acetic acid and 0.145 g of triethylamine in 15 ml of methylene chloride, and then the mixture was stirred for 2 hours at -20°C. The mixture was cooled to -70°C, and to the mixture were added 0.145 g of triethylamine, 0.095 g of 1-tert-butyldimethylsilyl-3-amino-2-oxoazetidine and 4 ml of propyleneoxide. The mixture was reacted for one hour, then the temperature returned to a room temperature.

The reaction mixture was subjected to concentration and purification with silica gel column chromatography to give 0.296 g of 1-tert-butyldimethylsilyl-3-[2-(2-chloro-acetamidothiazol-4-yl)-2-(2-trimethylsilylethoxyimino)-acetamide]-2-oxoazetidine.

IR $\nu_{max}^{KBr}cm^{-1}$: 1740, 1680, 1520

NMR($CDCl_3$, ppm): 0.24(s,$CH_3$), 0.98(s,t-Bu), 1.00(t,J=8Hz,$-CH_2-$), 1.60(s,$CH_3$), 3.40(dd,J=3,5Hz, $C_4$-H), 3.68(t,J=5Hz,$C_4$-H), 4.20(t,J=8Hz,$-CH_2-$), 4.25 (s,$-CH_2-$), 5.20(m,$C_3$-H), 6.50(s,NH), 7. 40(s, 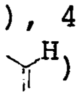), 8.05(d,J=8Hz,NH), 10.50(s,NH).

Example 18

A mixture of 335 mg of 1-tert-butyldimethylsilyl-3-benzyloxycarboxamido-2-oxoazetidine, 320 mg of pyridine-sulfuric anhydride complex and 2 ml of dimethylformamide was reacted at 25°C for 42 hours. Dimethylformamide was evaporated under reduced pressure, and then the residue was

washed with diethyl ether. After the addition of water, the insoluble was filtered off and the filtrate was passed through Dowex 50W (Dow Chemical Co., U.S.A.) $Na^+$ type column and, then, was chromatographed on a column of Amberlite XAD-II (Rohm and Haas Co., U.S.A.) to give 125 mg of sodium 3-benzyloxycarboxamido-2-oxoazetidine-1-sulfonate.

IR $\nu_{max}^{KBr}cm^{-1}$: 1760, 1690, 1540, 1260, 1240, 1055.

NMR(DMSO-$d_6$, ppm): 3.60(t,J=6Hz,$C_4$-H), 4.60(m,$C_3$-H), 5.0(s,$CH_2$), 7.23(s,ar.H), 7.90(d,J=8Hz,NH).

Example 19

A mixture of 364 mg of 1-tert-butyldimethylsilyl-3-benzyloxycarboxamido-3-methoxy-2-oxoazetidine, pyridine-sulfuric anhydride complex and 2 ml of dimethylformamide was stirred at 60°C for 16 hours and, then, the resultant was treated in the manner of Example 18 to give 150 mg of sodium 3-benzyloxycarboxamido-3-methoxy-2-oxoazetidine-1-sulfonate.

IR $\nu_{max}^{KBr}cm^{-1}$: 1760, 1720, 1625, 1505, 1260, 1050.

NMR(DMSO-$d_6$, ppm): 3.36(s,$CH_3$),3.56(d,J=6Hz,$C_4$-H), 3.76(d,J=6Hz,$C_4$-H), 5.13(s,-$CH_2$-), 7.40(s,ar.H), 8.90(broad s,NH).

Example 20

A mixture of 250 mg of 1-isopropyldimethylsilyl-3-phenylacetamido-2-oxoazetidine, 261 mg of pyridine-sulfuric anhydride complex and 2 ml of dimethylformamide was reacted at room temperature for 24 hours. The resultant was treated in the manner of Example 18 to give 120 mg of sodium 3-phenylacetamido-2-oxoazetidine-1-sulfonate.

IR $\nu_{max}^{KBr}cm^{-1}$: 3300, 1775, 1665, 1300-1190, 1045.

NMR(DMSO-$d_6$, ppm): 3.25(dd,J=3,6Hz,$C_4$-H), 3.46(s,-$CH_2$-), 3.62(t,J=6Hz,$C_4$-H), 4.84(ddd,J=3,6,8Hz,$C_3$-H), 7.29 (s,ar.H), 8.83(d,J=8Hz,NH).

Example 21

To a solution of 299 mg of 1-tert-butyldimethylsilyl-3-phenylacetamido-2-oxoazetidine in 3 ml of dimethylformamide, was added 2.26 ml of 1N dimethylformamide-sulfuric

anhydride complex dimethylformamide solution under cooling at -20°C, followed by reacting at -17 to -15°C for 24 hours. After adding 180 mg of pyridine, the resultant was treated in the manner of Example 18 to give 115.7 mg of sodium 3-phenylacetamido-3-methoxy-2-oxoazetidine-1-sulfonate.

Example 22

A mixture of 348 mg of 1-tert-butyldimethylsilyl-3-phenylacetamido-3-methoxy-2-oxoazetidine, 172 mg of pyridine-sulfuric anhydride complex, 58 mg of anhydrous potassium fluoride and 2 ml of dimethylformamide was reacted at room temperature for 16 hours. The resultant was treated in the manner of Example 18 to give 16.7 mg of sodium 3-phenylacetamido-3-methoxy-2-oxoazetidine-1-sulfonate.

IR $\nu_{max}^{KBr} cm^{-1}$: 1760, 1665, 1520, 1250.

NMR(DMSO-$d_6$, ppm): 3.40(s,$CH_3$), 3.56, 3.80(each d,J=6Hz, $C_4$-H), 3.60(s,-$CH_2$-), 7.30(s,ar.H), 8.90(broad s,NH).

Example 23

A solution of 210 mg of 1-isopropyldimethylsilyl-3-phenylacetamido-3-methoxy-2-oxoazetidine, 180 mg of pyridine-sulfuric anhydride complex and 2 ml of dimethylformamide was reacted at room temperature for 17 hours. The resultant was treated in the manner of Example 18 to give 10 mg of sodium 3-phenylacetamido-3-methoxy-2-oxoazetidine-1-sulfonate.

Example 24

A mixture of 520 mg of 1-tert-butyldimethylsilyl-3-[2-isopropoxyimino-2-(2-chloroacetamidothiazol-4-yl)-acetamido]-3-methoxy-2-oxoazetidine, 320 mg of pyridine-sulfuric anhydride complex and 4 ml of dimethylformamide was reacted at 25°C for 24 hours. The resultant was treated in the manner of Example 18 to give 246 mg of sodium 3-[2-isopropoxyimino-2-(2-chloroacetamidothiazol-4-yl)acetamido]-3-methoxy-2-oxoazetidine-1-sulfonate.

IR $\nu_{max}^{KBr} cm^{-1}$: 1765, 1675, 1540, 1240, 1050.

NMR(DMSO-$d_6$, ppm): 1.24, 1.26(each d,J=6Hz,$CH_3$), 3.45 (s,$CH_3$), 3.59, 3.85(each d,J=6Hz,$C_4$-H), 4.36

(quintet,J=6Hz,-CH<), 4.38(s,$-CH_2-$), 7.39(s, 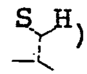), 9.84(s,NH).

A mixture of 200 mg of sodium 3-[2-isopropoxyimino-2-(2-chloroacetamidothiazol-4-yl)acetamido]-3-methoxy-2-oxoazetidine-1-sulfonate obtained as mentioned above, 51 mg of sodium N-methyldithiocarbamate and 5 ml of water was stirred at 20°C for 2 hours. The resultant was chromatographed on a column of Amberlite XAD-II to give 70 mg of 3-[2-isopropoxyimino-2-(2-aminothiazol-4-yl)acetamido]-3-methoxy-2-oxoazetidine-1-sulphonate.

IR $\nu_{max}^{KBr}cm^{-1}$: 1770, 1670, 1620, 1530, 1245, 1055.

NMR($DMSO-d_6$, ppm): 1.20, 1.22(each d,J=6Hz,$CH_3$), 3.41 (s,$CH_3$), 3.56, 3.80(each d,J=6Hz,$C_4$-H), 4.30(quintet, J=6Hz,-CH<), 6.67(s, 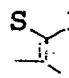), 7.11(broad s,$-NH_2$), 9.72(s,NH).

Example 25

A mixture of 385 mg of 1-tert-butyldimethylsilyl-3-[2-[1-(tert-butyloxycarbonyl)isopropoxyimino]-2-(2-tritylaminothiazol-4-yl)acetamide]-2-oxoazetidine and 160 mg of pyridine-sulfuric anhydride complex in 3 ml of dimethylformamide was reacted at 25°C for 48 hours. The resultant was treated in the manner of Example 18 to give two products, i.e. product (A): 70 mg of disodium 3-[2-[1-(tert-butyloxycarbonyl)isopropoxyimino]-2-(2-sulfonatoaminothiazol-4-yl)acetamido]-2-oxoazetidine-1-sulfonate and product (B): 55 mg of sodium 3-[2-[1-(tert-butyloxycarbonyl)isopropoxyimino]-2-(2-tritylaminothiazol-4-yl)-acetoamido]-2-oxoazetidine-1-sulfonate.

(A) IR $\nu_{max}^{KBr}cm^{-1}$: 1760, 1730, 1665, 1630, 1525, 1250, 1145, 1050.

NMR($DMSO-d_6$, ppm): 1.39(s,t-Bu), 1.40(s,$-C(CH_3)_2-$), 3.35 (m,$C_4$-H), 4.95(m,$C_3$-H), 6.83(s, 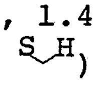), 9.10(d,J=8Hz,NH)

(B) IR $\nu_{max}^{KBr}cm^{-1}$: 1760, 1730, 1675, 1590, 1570, 1280, 1250, 1200, 1140, 1040.

NMR($DMSO-d_6$, ppm): 1.38, 1.40(each s,t-Bu), 3.35(m,

$C_4$-H), 4.90(m,$C_3$-H), 6.75, 6.96(each s, 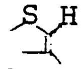), 7.23, 7.33(each s,ar.H), 9.03, 9.26(each d,J=9Hz,NH).

Example 26

0.48 g of 1-tert-butyldimethylsilyl-3-methoxy-3-[2-(2-methanesulfonaminothiazol-4-yl)-2-isopropoxyimino-acetamido]-2-oxoazetidine, 0.054 g of potassium fluoride, 0.263 g of pyridine-sulfuric anhydride complex was treated in the manner of Example 18 to give 0.043 g of sodium 3-methoxy-3-[2-(2-methanesulfonamidothiazol-4-yl)-2-isopropoxyiminoacetamido]-2-oxoazetidine-1-sulfonate.

IR $\nu_{max}^{KBr}cm^{-1}$: 3450, 3250, 1768, 1670, 1530, 1500, 1470, 1240, 1115, 1052.

NMR(DMSO-$d_6$, ppm): 1.22(d,J=6Hz,$CH_3$), 2.74(s,$CH_3$), 3.41 (s,$CH_3$), 3.68(ABq,J=6,21Hz,$C_4$-H), 4.37(heptet. J=6Hz,-CH-), 6.63(s, 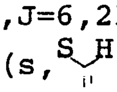), 9.80(s,NH).

Example 27

To a solution of 0.615 g of 1-tert-butyldimethylsilyl-3-[2-(2-chloroacetamidothiazol-4-yl)-2-[1-(2-trimethylsilylethoxycarbonyl)isopropoxyimino]acetamido]-2-oxoazetidine in 5 ml of dimethylformamide, was added a solution of dimethylformamide-sulfuric anhydride complex dissolved in 2.0 ml of dimethylformamide at -20°C. The mixture was reacted for 2 hours at 0°C. The resultant was treated in the manner of Example 18 to give 0.44 g of sodium 3-[2-(2-chloroacetamidothiazol-4-yl)-2-[1-(2-trimethylsilylethoxycarbonyl)isopropoxyimino)acetamido]-2-oxoazetidine-1-sulfonate.

IR $\nu_{max}^{KBr}cm^{-1}$: 3450, 3270, 1755, 1662, 1538, 1270, 1245, 1145, 1048.

NMR(DMSO-$d_6$, ppm): 0.03(s,$CH_3$), 0.96(t,J=8Hz,-$CH_2$-), 1.47(s,$CH_3$), 3.31(dd,J=3,6Hz,$C_4$-H), 3.68(t,J=6Hz, $C_3$-H), 4.17(t,J=8Hz,-$CH_2$-), 4.35(s,-$CH_2$-), 4.98(m, $C_3$-H), 7.38(s, 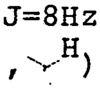), 9.14(d,J=8Hz,NH), 12.83(broad s,NH).

In accordance with the procedure of Examples 1, 2, 18 and 19, the following compound are produced.

28. Sodium 3-(α-sulfophenylacetamido)-2-oxoazetidine-1-sulfonate
29. Sodium 3-[2-(4-ethyl-2,3-dioxo-1-piperazinecarboxamido)-2-phenylacetamido]-2-oxoazetidine-1-sulfonate
30. Sodium 3-[2-(benzyloxycarboxamido)-2-phenylacetamido]-2-oxoazetidine-1-sulfonate
31. Sodium 3-[2-(2-chloroacetamidothiazol-4-yl)-2-methoxyiminoacetamido]-3-methoxy-2-oxoazetidine-1-sulfonate (syn-isomer)
    (by the deprotection of the above-mentioned compound with sodium N-methyldithiocarbamate, sodium 3-[2-(2-aminothiazol-4-yl)-2-methoxyiminoacetamido]-3-methoxy-2-oxoazetidine-1-sulfonate(syn-isomer) is obtained)
32. Sodium 3-cyanoacetamido-2-oxoazetidine-1-sulfonate
33. Sodium 3-(1H-tetrazole-1-acetamido)-2-oxoazetidine-1-sulfonate
34. Sodium 3-[3-(2,6-dichlorophenyl)-5-methylisoxazol-4-yl]carboxamido-2-oxoazetidine-1-sulfonate
35. Sodium 3-benzyloxycarboxamido-2-oxoazetidine-1-sulfonate
36. Sodium 3-(N-carbobenzoxy-D-alaninamido)-2-oxoazetidine-1-sulfonate.
    (To the above-mentioned compound, are added 20 mg of acetic acid and 50 mg of palladium black and the mixture is stirred in a stream of hydrogen for 20 minutes. The catalyst is filtered off and the filtrate is lyophilized to give acetate of 3-D-alaninamido-2-oxoazetidine-1-sulfonate
37. Sodium 3-(α-benzyl-N-carbobenzoxy-γ-D-glutamyl-D-alaninamido)-2-oxoazetidine-1-sulfonate
    (The above-mentioned compound is passed through Dowex 50W-Na type resin and the eluant is lyophilized and, then, is dissolved to 20 ml of water. To the solution, is added 0.20 g of palladium black and the mixture is stirred in stream of hydrogen for an hour. The catalyst is filtered off and the filtrate is lyophilized to give sodium 3-(γ-D-glutamyl-D-alaninamido)-

2-oxoazetidine-1-sulfonate.)

38. Sodium 3-(α-ureidophenylacetamido)-2-oxoazetidine-1-sulfonate
39. Disodium 3-(α-sulfonatoureidophenylacetamido)-2-oxoazetidine-1-sulfonate
40. Sodium 3-[2-(4-ethyl-2,3-dioxo-1-piperazinecarboxamido)-2-phenylacetamido]-3-methoxy-2-oxoazetidine-1-sulfonate
41. Disodium 3-[2-(4-ethyl-2,3-dioxo-1-piperazinecarboxamido)-2-(4-hydroxysulfonyloxyphenyl)acetamido]-2-oxoazetidine-1-sulfonate
42. Sodium 3-[2-(4-ethyl-2,3-dioxo-1-piperazinecarboxamido)-2-(4-hydroxyphenyl)acetamido]-2-oxoazetidine-1-sulfonate
43. Sodium 3-(4-ethyl-2,3-dioxo-1-piperazinecarboxamido)-2-oxoazetidine-1-sulfonate
44. Sodium 3-[N-(4-ethyl-2,3-dioxo-1-piperazinecarbonyl)-D-alaninamido]-2-oxoazetidine-1-sulfonate
45. Disodium 3-(α-hydroxysulfonyloxyphenylacetamido)-2-oxoazetidine-1-sulfonate
46. Sodium 3-(2-syn-methoxyimino-2-phenylacetamido)-2-oxoazetidine-1-sulfonate
47. Sodium 3-amino-3-methoxy-2-oxoazetidine-1-sulfonate
48. 3-Thienylacetamido-2-oxoazetidine-1-sulfonic acid
49. Sodium 3-[2-(2-aminothiazol-4-yl)-2-methoxyimino-acetamido]-2-oxoazetidine-1-sulfonate (syn-isomer)
50. Sodium 3-[2-(2-aminothiazol-4-yl)acetmaido]-2-oxo-azetidine-1-sulfonate
51. Disodium 3-(α-sulfophenylacetamido)-2-oxoazetidine-1-sulfonate
52. Sodium 3-[2-(2-aminothiazol-4-yl)-2-methoxyimino-acetamido]-3-methoxy-2-oxoazetidine-1-sulfonate(syn-isomer)
53. Sodium 3-D-alaninamido-2-oxoazetidine-1-sulfonate· acetic acid salt.
54. Sodium 3-[D-2-(4-ethyl-2,3-dioxo-1-piperazinecarboxamido)-2-(4-methoxyphenyl)acetamido]-2-oxoazetidine-1-sulfonate

55. Sodium 3-[D-2-[2-(2-aminothiazol-4-yl)-2-methoxyimino-acetamido]-2-phenylacetamido]-2-oxoazetidine-1-sulfonate
56. Sodium 3-[D-2-(6-bromo-1,4-dihydro-1-ethyl-4-oxo-thieno[2,3-b]pyridine-3-carboxamido)-2-phenylacetamido]-2-oxoazetidine-1-sulfonate
57. Sodium 3-[2-(4-ethyl-2,3-dioxo-1-piperazinecarboxamido)-2-(2-acetamidothiazol-4-yl)acetamido]-2-oxoazetidine-1-sulfonate
58. Sodium 3-[2-(2-aminothiazol-4-yl)-2-methoxyiminoacetamido]-2-(2-aminothiazol-4-yl)acetamido]-2-oxoazetidine-1-sulfonate(syn-isomer)
59. Sodium 3-[D-2-(4-n-octyl-2,3-dioxo-1-piperazinecarboxamido)-2-phenylacetamido]-2-oxoazetidine-1-sulfonate
60. Sodium 3-[D-2-(coumarine-3-carboxamido)-2-phenylacetamido)-2-oxoazetidine-1-sulfonate
61. Sodium 3-[2-(4-n-octyl-2,3-dioxo-1-piperazinecarboxamido)-2-(2-aminothiazol-4-yl)acetamido]-2-oxoazetidine-1-sulfonate
62. Sodium 3-[D-2-(4-hydroxy-7-trifluoromethylquinoline-3-carboxamido)-2-phenylacetamido]-2-oxoazetidine-1-sulfonate
63. Sodium 3-[D-2-(4-n-octyl-2,3-dioxo-1-pierazinecarboxamido)-2-(4-hydroxyphenyl)acetamido]-2-oxoazetidine-1-sulfonate and disodium 3-[D-2-(4-n-octyl-2,3-dioxo-1-piperadinecarboxamido)-2-(4-hydroxysulfonyloxyphenyl)-acetamido]-2-oxoazetidine-1-sulfonate
64. Sodium 3-[2-(2-oxo-3-furfurylideneaminoimidazolidin-1-yl)carboxamido]-2-thienylacetamido]-2-oxoazetidine-1-sulfonate
65. Sodium 3-[2-(4-ethyl-2,3-dioxo-1-pierazinecarboxamido)-2-(2-pyrrolyl)acetamido]-2-oxoazetidine-1-sulfonate
66. Sodium 3-[D-2-(4-n-octyl-2,3-dioxo-1-pierazinecarboxamido]-2-thienylacetamido]-3(R)-methoxy-2-oxoazetidine-1-sulfonate and sodium 3-[L-2-(4-n-octyl-2,3-dioxo-1-piperazinecarboxamido)-2-thienylacetamido]-3(S)-methoxy-2-oxoazetidine-1-sulfonate

67. Disodium 3-(D-α-sulfophenylacetamido)-3(R)-methoxy-2-oxoazetidine-1-sulfonate and disodium 3-(D-α-sulfophenylacetamido)-3(S)-methoxy-2-oxoazetidine-1-sulfonate
68. Sodium 3-(N-benzyloxycarbonyl-D-alanyl-D-phenylglycylamino)-2-oxoazetidine-1-sulfonate
Sodium 3-(D-alanyl-D-phenylglycylamino)-2-oxoazetidine-1-sulfonate
69. Pyridium 3-[D-2-(2-ureido-2-thienylacetamido)-2-phenylacetamido]-2-oxoazetidine-1-sulfonate
70. Sodium 3-[D-2-[2-(2-aminothiazol-4-yl)acetamido]-2-phenylacetamido]-2-oxoazetidine-1-sulfonate
71. A) Sodium 3-[DL-2-(4-ethyl-2,3-dioxo-1-piperadinecarboxamido)-2-thienylacetamido]-2-oxoazetidine-1-sulfonate
B) Sodium 3-[D-2-(4-ethyl-2,3-dioxo-1-piperazinecarboxamido)-2-thienylacetamido]-2-oxoazetidine-1-sulfonate
C) Sodium 3-[L-2-(4-ethyl-2,3-dioxo-1-piperadinecarboxamido)-2-thienylacetamido]-2-oxoazetidine-1-sulfonate
72. Sodium 3-(2-thienyl-2-methoxyiminoacetamido)-2-oxoazetidine-1-sulfonate
73. Sodium 3-[2-thienyl-2-(3-morpholinopropyloxyimino)-acetamido]-2-oxoazetidine-1-sulfonate
74. Sodium 3-[D-2-[DL-2-(4-ethyl-2,3-dioxo-1-piperazinecarboxamido)-2-thienylacetamido]-2-phenylacetamido]-2-oxoazetidine-1-sulfonate
75. Sodium 3-[2-(3,5-dioxo-1,2,4-triazine-6-carboxamido)-2-thienylacetamido]-2-oxoazetidine-1-sulfonate
76. Sodium 3-[2-(4-ethyl-2,3-dioxo-1-piperazinecarboxamido)-2-(2-methylthiazol-4-yl)acetamido]-2-oxoazetidine-1-sulfonate
77. Sodium 3-[2-(4-chlorobenzoylureido)-2-thienylacetamido]-2-oxoazetidine-1-sulfonate
78. Sodium 3-cyanomethylthioacetamido-3-methoxy-2-oxoazetidine-1-sulfonate

79. Sodium 3-(2-carboxy-2-phenylacetamido)-3-methoxy-2-oxoazetidine-1-sulfonate
80. Sodium 3-[2-(5,6-dihydro-1,4-oxathiin-2-yl)acetamido]-2-oxoazetidine-1-sulfonate
81. Sodium 3-(D-carbamoyltriptophyl-D-phenylglycylamino)-2-oxoazetidine-1-sulfonate
82. Sodium 3-[D-N-(4-ethyl-2,3-dioxo-1-piperazinecarbonyl)-phenylalanylamino]-2-oxoazetidine-1-sulfonate
83. Sodium 3-[2-(2,4-dioxopyrimidino-5-carboxamido)-2-thienylacetamido]-2-oxoazetidine-1-sulfonate
84. Disodium 3-[D-2-(2-ureido-2-thienylacetamido)-2-(4-hydroxysulfonyloxyphenyl)acetamido]-2-oxoazetidine-1-sulfonate and sodium 3-[D-2-(2-ureido-2-thienylacetamido)-2-(4-hydroxyphenyl)acetamido]-2-oxoazetidine-1-sulfonate
85. Sodium 3-[D-N-(4-ethyl-2,3-dioxo-1-piperazinocarbonyl)-α-glutamylamino]-2-oxoazetidine-1-sulfonate
86. Sodium 3-[2-(4-ethyl-2,3-dioxo-1-piperazinecarboxamido)-2-(1-cyclohexen-1-yl)acetamido]-2-oxoazetidine-1-sulfonate.
87. Sodium 3-[2-(4-ethyl-2,3-dioxo-1-piperazinecarboxamido)-2-(4-chlorophenyl)acetamido]-2-oxoazetidine-1-sulfonate
88. Sodium 3-[2-(4-ethyl-2,3-dioxo-1-piperazinecarboxamido)-2-(4-trimethylsilylphenyl)acetamido]-2-oxo-azetidine-1-sulfonate
89. Sodium 3-[D-N-(4-ethyl-2,3-dioxo-1-piperazinecarbonyl)-methionyl-D-phenylglycylamino]-2-oxoazetidine-1-sulfonate
90. Sodium 3-[2-(3-methylcarbamoyl-3-methyl-1-ureido)-2-thienylacetamido]-2-oxoazetidine-1-sulfonate
91. Sodium 3-[D-2-(3-chloro-4-methoxyphenyl)-2-(4-ethyl-2,3-dioxo-1-piperazinecarboxamido)acetamido]-2-oxo-azetidine-1-sulfonate
92. Sodium 3-[D-2-(2-amino-3-N-methylcarbamoylpropionamido)-2-phenylacetamido]-2-oxoazetidine-1-sulfonate
93. Sodium 3-[D-2-(3-amino-3-N-methylcarbamoylpropionamido)-2-phenylacetamido]-2-oxoazetidine-1-sulfonate

94. Sodium 3-[2-(2,5-dioxopyrolidin-3-yl)acetamido]-2-oxoazetidine-1-sulfonate
95. Sodium 3-(2-succinimidoacetamido)-2-oxoazetidine-1-sulfonate
96. Sodium 3-(2-methoxyimino-2-furylacetamido)-2-oxo-azetidine-1-sulfonate
97. Sodium 3-[2-(2-ureidomethylphenyl)acetamido]-2-oxoazetidine-1-sulfonate
98. Sodium 3-[2-(3,5-dichloro-pyridon-1-yl)acetamido]-2-oxoazetidine-1-sulfonate
99. Sodium 3-[2-phenyl-2-benzyloxycarbonylacetamido)-2-oxoazetidine-1-sulfonate
100. Sodium 3-[2-(N-carbobenzoxyprolylamino)-2-furyl-acetamido]-2-oxoazetidine-1-sulfonate
101. Sodium 3-[2-(1-acetyl-2,4-dioxoimidazolidin-3-yl)-acetamido]oxoazetidine-1-sulfonate
102. Sodium 3-[2-(2-oxoimidazolidin-1-yl)acetamido]-2-oxoazetidine-1-sulfonate
103. Sodium 3-[2-[2-(2-oxoimidazolidin-1-yl)carbonylamino-methylphenyl]acetamido]-2-oxoazetidine-1-sulfonate
104. Sodium 3-[2-[2-(2-oxo-3-benzylideneaminoimidazolidin-1-yl)carboxyaminomethylphenyl]acetamido]-2-oxoazeti-dine-1-sulfonate
105. Sodium 3-[2-(4-ethyl-2,3-dioxo-1-piperazinecarboxamido)-2-furylacetamido]-2-oxoazetidine-1-sulfonate
106. Sodium 3-(N-carbobenzoxy-D-alaninamido)-3-methoxy-2-oxoazetidine-1-sulfonate
107. Sodium 3-[(N-carbobenzoxy-D-phenylglycyl)-D-phenyl-glycinamido]-3-methoxy-2-oxoazetidine-1-sulfonate
108. Sodium 3-[N-(4-ethyl-2,3-dioxo-1-piperazinecarbonyl)-D-methioninamido]-2-oxoazetidine-1-sulfonate
109. Sodium 3-[2-D-[4-(2-phenethyl)-2,3-dioxo-1-piperazine-carboxamido]-2-phenylacetamido]-2-oxoazetidine-1-sulfonate
110. Sodium 3-[2-D-(4-ethyl-2,3-dioxo-1-piperazinecarbox-amido)-2-(4-benzoyloxyphenyl)acetamido]-2-oxoazetidine-

sulfonate

111. Sodium 3-[2-benzyloxycarboxamido-3-(N-methylcarbamoyl)-propionamido]-3-methoxy-2-oxoazetidine-1-sulfonate

112. Sodium 3-[2-(3-furfurylideneamino-2-oxoimidazolidin-1-yl)carboxamido-2-(2-aminothiazol-4-yl)acetamido]-2-oxoazetidine-1-sulfonate

113. Sodium 3-[D-2-(2-phenylacetamido)propionamido]-3-methoxy-2-oxoazetidine-1-sulfonate

114. Sodium 3-[2-([2-oxo-3-(thiophen-2-aldoimino)imidazolidin-1-yl]carboxamido]-2-thienylacetamido]-2-oxoazetidine-1-sulfonate

115. Sodium 3-[2-[(3-mesyl-2-oxoimidazolidin-1-yl)carboxamido]-2-thienylacetamido]-2-oxoazetidine-1-sulfonate

116. Sodium 3-[D-2-(2,6-dichlorophenylthioglycolamido)-2-phenylacetamido]-2-oxoazetidine-1-sulfonate

117. Sodium 3-[(hexahydro-1H-azepin-1-yl)methyleneamino)]-2-oxoazetidine-1-sulfonate

118. Sodium 3-(2-oxyimino-2-thienylacetamido)-2-oxoazetidine-1-sulfonate

119. Sodium 3-(2-phenyl-2-sulfamoylacetamido)-2-oxoazetidine-1-sulfonate

120. Sodium 3-(2-N,N-dimethylsulfamoyl-2-phenylacetamido)-2-oxoazetidine-1-sulfonate

121. Sodium 3-[2,5-(bis(4-ethyl-2,3-dioxo-1-piperazinecarboxamido)pentanamido]-2-oxoazetidine-1-sulfonate

122. Sodium 3-[D-2-[4-(2-chloromethyl)-2,3-dioxo-1-piperazinecarboxamido]-2-phenylacetamido]-2-oxoazetidine-1-sulfonate

123. Sodium 3-[D-2-chloro-2-(4-ethyl-2,3-dioxo-1-piperazinecarboxamido)propionamido]-2-oxoazetidine-1-sulfonate

124. Sodium 3-(2-benzyloxycarboxamido-2-benzyloxycarbonylethanesulfonamido)-2-oxoazetidine-1-sulfonate and sodium 3-(2-amino-2-carboxyethanesulfonamido)-2-oxoazetidine-1-sulfonate

125. Sodium 3-[D-2-(4-ethyl-2,3-dioxo-1-piperazinecarboxamido)-3-[(1-methyl-5H-tetrazol-5-yl)thio]propionamido]-

2-oxoazetidine-1-sulfonate

126. Sodium 3-[D-2-(2-amino-2-carboxyethanesulfonamido)-2-phenylacetamido]-2-oxoazetidine-1-sulfonate

127. Sodium 3-[D-2-(2-benzyloxycarboxamido-3-sulfamoyl propionamido)-2-phenylacetamido]-2-oxoazetidine-1-sulfonate

128. Sodium 3-[D-2-[2-amino-3-(4-ethyl-2,3-dioxo-1-piperazinecarboxamido)propionamido]-2-phenylacetamido]-2-oxoazetidine-1-sulfonate

129. Sodium 3-[2-(2-benzyloxycarboxamido-3-N-methyl-carbamoylpropionamido)acetamido]-3-methoxy-2-oxo-azetidine-1-sulfonate

130. Sodium 3-[D-2-[2-(4-ethyl-2,3-dioxo-1-piperazinecarboxamido)-3-(N-methylcarbamoyl)propionamido]-2-phenylacetoamido]-2-oxoazetidine-1-sulfonate

131. Sodium 3-[2-(4-ethyl-2,3-dioxo-1-piperazinecarboxamido)-3-(N-methylcarbamoyl)propionamido]-2-oxoazetidine-1-sulfonate

132. Sodium 3-[2-(D-2-amino-2-phenylacetamido-2-(N-methylcarbamoyl)propionamido]-2-oxoazetidine-1-sulfonate

133. Sodium 3-[2-(4-ethyl-2,3-dioxo-1-piperazinecarboxamido)-2-(4-n-octanoyloxyphenyl)acetamido]-2-oxoazetidine-1-sulfonate

134. Sodium 3-[D-2-(4-ethyl-2,3-dioxo-1-piperazinecarboxamido)-3-(2-thienylacetamido)propionamido]-2-oxoazetidine-1-sulfonate

135. Sodium 3-[D-2-[2-(4-ethyl-2,3-dioxo-1-piperazine-carboxamido)acetamido]-2-phenylacetamido]-2-oxoazetidine-1-sulfonate

136. Sodium 3-[D-2-[(3-furfurylideneamino-2-oxoimidazolidin-1-yl)carboxamido]propionamido]-2-oxoazetidine-1-sulfonate

137. Sodium 3-[D-2-[(3-furfurylideneamino-2-oxoimidazolidin-1-yl)carboxamido]propionamido]-3-methoxy-2-oxoazetidine-1-sulfonate

138. Sodium 3-[D-2-(4-n-octyl-2,3-dioxo-1-piperazinecarboxamido)-2-thienylacetamido]-2-oxoazetidine-1-sulfonate

139. Sodium 3-[D-2-(4-n-amyl-2,3-dioxo-1-piperazinecarboxamido)-2-thienylacetamido]-2-oxoazetidine-1-sulfonate

140. Sodium 3-[2-(5-chloro-2-chloroacetylamino-oxazol-4-yl)-2-methoxyiminoacetamido]-2-oxoazetidine-1-sulfonate
(The above-mentioned compound is reacted with sodium N-methyldithiocarbamate in water to give sodium 3-[2-(2-amino-5-chlorothiazol-4-yl)-2-methoxyiminoacetamido]-2-oxoazetidine-1-sulfonate.)

141. Sodium 3-[D-2-(4,6-diethyl-2,3-dioxo-1-piperazinecarboxamido)-2-phenylacetamido]-2-oxoazetidine-1-sulfonate

142. Sodium 3-(2-phenyl-2-p-tolylthioiminoacetamido)-2-oxoazetidine-1-sulfonate

143. Sodium 3-(2,6-dimethoxybenzamido)-2-oxoazetidine-1-sulfonate

144. Sodium 3-[D-2-(4-n-amyl-6-methyl-2,3-dioxo-1-piperazinecarboxamido)-2-thienylacetamido]-2-oxoazetidine-1-sulfonate

145. Sodium 3-[D-2-(4-n-amyl-6-methyl-2,3-dioxo-1-piperazinecarboxamido)-3-chloropropionamido]-2-oxoazetidine-1-sulfonate

146. Sodium 3-[D-2-[[3-(2,6-dichlorophenyl)-5-methylisoxazol-4-yl]carboxamido]-2-thienylacetamido]-2-oxoazetidine-1-sulfonate

147. Sodium 3-[D-2-(4-ethyl-5-methyl-2,3-dioxo-1-piperazinecarboxamido)-2-thienylacetamido]-2-oxoazetidine-1-sulfonate

148. Disodium 3-[D-2-[(2-oxo-3-sulfonatoimidazolidin-1-yl)-carboxamido]-2-thienylacetamido]-2-oxoazetidine-1-sulfonate

149. Sodium 3-[D-2-[(2-oxoimidazolidin-1-yl)carboxamido]-

2-thienylacetamido]-2-oxoazetidine-1-sulfonate

150. Sodium 3-[D-2-[(5-benzyloxycarbonyl-3-methyl-2-oxo-imidazolidin-1-yl)carboxamido]-2-phenylacetamido]-2-oxoazetidine-1-sulfonate
(The above-mentioned compound in water is worked up in a stream of hydrogen at the presence of palladium black to obtain sodium 3-[D-2-[(5-carboxy-3-methyl-2-oxoimidazolidin-1-yl)carboxamido]-2-phenylacetamido]-2-oxoazetidine-1-sulfonate.

151. Sodium 3-[2-(4-ethyl-2,3-dioxo-1-piperazinecarboxamido)-2-(2-chloro-1-cyclohexen-1-yl)acetamido]-2-oxoazetidine-1-sulfonate

152. Sodium 3-[2-(2-amionthiazol-4-yl)-2-isopropoxyimino-acetamido]-2-oxoazetidine-1-sulfonate

153. Sodium 3-(2-oxo-2-phenylacetamido)-2-oxoazetidine-1-sulfonate

154. Sodium 3-(2-bromo-2-phenylacetamido)-2-oxoazetidine-1-sulfonate

155. Sodium 3-(2-phthalimido-2-thienylacetamido)-2-oxo-azetidine-1-sulfonate

156. Sodium 3-[2-azido-2-(3-chlorophenyl)acetamido)-2-oxoazetidine-1-sulfonate

157. Sodium 3-(2-azido-2-phenylacetamido)-3-methoxy-2-oxoazetidine-1-sulfonate

158. Sodium 3-[D-2-[4-(2-hydroxyethyl)-2,3-dioxo-1-piperazinocarboxamido]-2-phenylacetamido]-2-oxo-azetidine-1-sulfonate

159. Sodium 3-[2-(4-ethyl-2,3-dioxo-1-piperazinecarboxamido)-2-(benzo[b]thiophen-3-yl)acetamido]-2-oxoazetidine-1-sulfonate

160. Sodium 3-[D-α-N-(L-N-carbobenzoxy-2-sulfamoylalanyl)-alaninamido]-3-methoxy-2-oxoazetidine-1-sulfonate

161. Sodium 3-[2-(4-ethyl-2,3-dioxo-1-piperazinecarbox-amido)-2-(3,5-dimethylpyrazol-4-yl)acetamido]-2-oxo-azetidine-1-sulfonate

162. Sodium 3-[2-(2-amino-3-fluoropropionamide)-3-fluoropro-

pionamido]-2-oxoazetidine-1-sulfonate

163. Sodium 3-[D-2-[5-isobutyl-4-ethyl-2,3-dioxo-1-piperazinecarboxamido]-2-thienylacetamido]-2-oxo-azetidine-1-sulfonate

164. Sodium 3-[D-2-[4-(3-methylbut-2-en-1-yl)-2,3-dioxo-1-piperazinecarboxamido]-2-thienylacetamido]-2-oxo-azetidine-1-sulfonate

165. Sodium 3-[D-2-(4-formylmethyl-2,3-dioxo-1-piperazine-carboxamido)-2-phenylacetamido]-2-oxoazetidine-1-sulfonate

166. Sodium 3-[D-2-[4-(2-methoxyiminoethyl)-2,3-dioxo-1-piperazinecarboxamido]-2-phenylacetamido]-2-oxo-azetidine-1-sulfonate

167. Sodium 3-[3-(2-methoxyethylidene)-7-oxo-4-oxa-1-azabicyclo(3,2,0)heptan-2-carboxamido]-2-oxoazetidine-1-sulfonate

168. Sodium 3-[D-2-[4-(1-piperazinocarbonylmethyl)-2,3-dioxo-1-piperazinecarboxamido]-2-thienylacetamido]-2-oxoazetidine-1-sulfonate

169. Disodium 3-[D-2-[4-[2-(4,4-dimethyl-3-sulfonatoiso-thiosemicarbazono)ethyl]-2,3-dioxo-1-piperazine-carboxamido]-2-phenylacetamido]-2-oxoazetidine-1-sulfonate

170. Sodium 3-[D-2-[4-(tetrahydrofurfuryl-2-ylmethyl)-2,3-dioxo-1-piperazinecarboxamido]-2-thienylacetamido]-2-oxoazetidine-1-sulfonate

171. Sodium 3-[D-2-[4-cyclohexyl-5-methyl-2,3-dioxopipera-zinecarboxamido]-2-thienylacetamido]-2-oxoazetidine-1-sulfonate.

172 Sodium 3-[D-2-(4-benzhydryl-2,3-dioxo-1-piperazinecarboxamido)-2-thienylacetamido]-2-oxoazetidine-1-sulfonate

173. Sodium 3-[D-2-(3,4-dioxohexahydropyrido[1,2-a]pyrazin-2-ylcarboxamido)-2-thienylacetamido]-2-oxoazetidine-1-sulfonate

What is claimed is:

1. A method of preparing 1-sulfo-2-oxoazetidine derivatives represented by the formula:

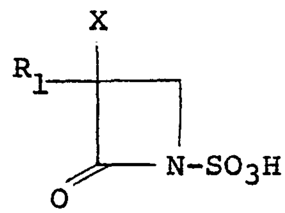

(I)

wherein $R_1$ stands for an amino group which may optionally be acylated or protected, and X stands for hydrogen or methoxy, which comprises by subjecing a 1-t-butyl or isopropylsilyl derivative of a compound represented by the formula:

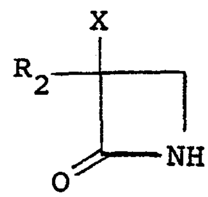

(II)

wherein $R_2$ stands for an amino group which is acylated or protected, and X is of the same meaning as defined above, to sulfonation, followed by, when $R_2$ is a protected amino group, removing the protective group, if necessary.

2. A method as claimed in Claim 1, wherein the sulfonation is carried out by employing sulfuric anhydride or a reactive derivative of sulfuric anhydride.

3. A method as claimed in Claim 2, wherein the reactive derivative of sulfuric anhydride is sulfuric anhydride-pyridine or sulfuric anhydride-N,N-dimethylformamide.

4. A method of preparing 1-sulfo-2-oxoazetidine derivatives respected by the formula:

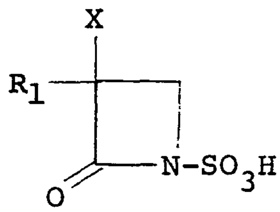

(I)

wherein $R_1$ stands for an amino group which may optionally be acylated or protected, and X stands for hydrogen or methoxy, which comprises reacting a compound of the formula:

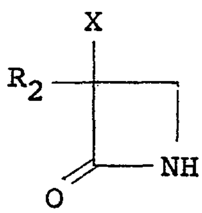

(II)

wherein $R_2$ stands for an amino group which is acylated or protected, and X is of the same meaning as defined above, with a t-butyl or isopropylsilyl compound to give a 1-t-butyl or isopropylsilyl derivative of the compound (II), and then, when $R_2$ is an acylated amino group, subjecting the 1-t-butyl or isopropylsilyl derivatives of the compound (II) to sulfonation,
when $R_1$ is a protected amino group, removing the protective group, acylating the amino group and subjecting the 1-t-butyl or isopropylsilyl derivative of the compound (II) to sulfonation,
or when $R_1$ is a protective amino group, subjecting the 1-t-butyl or isopropylsilyl derivative of the compound (II) to sulfonation without removing the protective group, followed by removing the protective group, if necessary.

5. A method as claimed in Claim 4, wherein the t-butyl or isopropylsilyl compound is tert-butyldimethylchlorosilane or isopropyldimethylchlorosilane.

6. A method as claimed in Claim 4, wherein the sulfonation is carried out by employing sulfuric anhydride or a reactive

derivative of sulfuric anhydride.

7. 1-t-Butyl or isopropylsilyl derivatives of a compound of the formula:

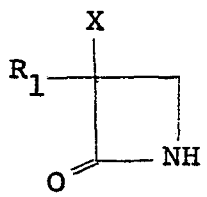

(II)

wherein $R_1$ stands for an amino group which may optionally be acylated or protected, and X stands for hydrogen or methoxy.

8. A compound as claimed in Claim 7 wherein the group $R_1$ is

(a) amino group, or

(b) acylated amino group, the acyl group being

(1) a group of the formula:

$R_6$-CO-

wherein $R_6$ is a lower alkyl group, phenyl which may optionally be substituted, benzoyl which may optionally be substituted, or a heterocyclic group which may optinoally be substitued,

(2) a group of the formula:

$$R_7\text{-NH-}\underset{\displaystyle R_9}{\underset{|}{\text{CH}}}\text{-CO-}$$

wherein $R_7$ is hydrogen, an amino acid residue which may optionally be substituted, an amino-protecting group, a group of $R_8$-$(CH_2)_n$-CO- [where $R_8$ is a heterocyclic group which may optionally be substituted, phenyl which may optionally be substituted, a lower alkyl group which may optionally be substitued, phenylthio which may optionally be substituted or a lower alkylthio group; n is an interger of 0 to 4; and the $-(CH_2)_n-$ group may be substituted], a group of $\genfrac{}{}{0pt}{}{R_8'}{R_8''}$N-CO- [where $R_8'$ and $R_8''$ may be the same or different and each is hydrogen, a lower alkyl group,

a lower alkylcarbamoyl group, phenylcarbonyl which may optionally be substituted, or sulfo] or a group of $R_8'''$ $-SO_2-$ [where $R_8'''$ is a lower alkyl group which may optionally be substituted]; $R_9$ is hydrogen, a lower alkyl group which may optionally be substituted, a phenyl group which may optionally be substituted, a heterocyclic group which may optionally be substituted, a cycloalkenylene group, a heterocycle-carbonylamino group which may optionally be substituted or be interrupted by an alkylene group,

(3) a group of the formula:

$$R_{10}-R_{11}-CO-$$

wherein $R_{10}$ is a group of the formula: $R_{12}-\underset{\underset{N-X'-R_{13}}{\|}}{C}-$ {where X' is oxygen or sulfur, $R_{12}$ is a heterocyclic group which may optionally be substituted or phenyl which may optionally be substituted, $R_{13}$ is hydrogen, phenyl which may optionally be substituted, a lower acyl group which may optionally be substituted, a lower alkyl group, or a group of the formula $-R_{14}-R_{15}$ (where $R_{14}$ is a lower alkylene or a lower alkenylene group, $R_{15}$ is carboxyl, an ester thereof or a heterocyclic group)}, $R_{11}$ is a single bond or a group of the formula $-CO-NH-\underset{\underset{R_{16}}{|}}{CH}-$ (where $R_{16}$ is a lower alkyl group, phenyl which may optionally be substituted or a heterocyclic group which may optionally be substituted),

(4) a group of the formula:

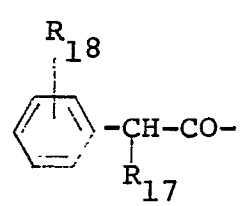

wherein $R_{17}$ is hydroxyl, hydroxysulfonyloxy, carboxyl, sulfamoyl which may optionally be substitued, sulfo, phenoxycarbonyl which may optionally be substituted, benzyloxycarbonyl, formyloxy, or phthalimide group; $R_{18}$

is hydrogen, a lower alkyl group, a lower alkoxy group, halogen, azide, nitro or hydroxyl, or

(5) a group of the formula:

$R_{19}-R_{20}-CH_2-CO-$

wherein $R_{19}$ is cyano, a phenyl group which may optionally be substitued, phenoxy which may optionally be substituted, a lower alkyl group which may optionally be substituted, an alkenylene group which may optionally be substituted, or a heterocyclic group which may optionally be substituted,

$R_{20}$ is a single bond or -S-, or

(c) aminoprotecting group selected from the group consisting of phthaloyl, p-nitrobenzoyl, p-tert-butylbenzoyl, p-tert-butylbenzenesulfonyl, benzenesulfonyl, toluenesulfonyl, formyl, acetyl, propionyl, monochloroacetyl, dichloroacetyl, trichloroacetyl, methanesulfonyl, ethanesulfonyl, trifluoroacetyl, maloyl, succinyl, methoxycarbonyl, ethoxycarbonyl, t-butoxycarbonyl, isopropoxycarbonyl, 2-cyanoethoxycarbonyl, β,β,β-trichloroethoxycarbonyl, benzyloxycarbonyl, p-nitrobenzyloxycarbonyl, p-methoxybenzyloxycarbonyl, diphenylmethyloxycarbonyl, methoxymethyloxycarbonyl, acetylmethyloxycarbonyl, isobornyloxycarbonyl, phenyloxocarbonyl, (hexahydro-1H-azepin-1-yl)methylene, 2-amino-2-carboxyethylsulfonyl, trityl, 2-nitrophenylthio, benzylidene, 4-nitrobenzylidene, di- or trialkylsilyl, benzyl and p-nitrobenzyl.

9. A compound as claimed in Claim 8, wherein the group $R_1$ is acylated amino group represneted by the formula:

A-B-CONH-

wherein A is hydrogen, a lower alkyl group, an alicyclic group, an aryl group, an aralkyl group or a heterocyclic group, these groups being unsubstituted or substituted,

B is (1) a group represented by the formula $\underset{W\quad Z}{-C-}$ wherein Z is hydrogen and W is optionally esterified carboxyl group, a sulfo group, a sulfamoyl group, a hydroxysulfonyloxy group, an optionally protected amino group, an amide, lower

alkylcarboxamide group or a heterocycle-carboxamide group, (2) a group of the formula =N-X'-G (in which X' is oxygen or sulfur atom or a sulfoxide group and G is a lower alkyl group, a carboxyl(lower)alkyl group, an aryl group or an acyl group, or (3) a single bond or (4) $-\overset{\overset{O}{\|}}{C}-$ .

10. A method of preparing a 1-t-butyl or isopropylsilyl derivatives of a compound of the formula:

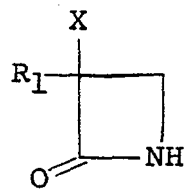

(I)

wherein $R_1$ stands for an amino group which may optionally be acylated or protected, and X stands for hydrogen or methoxy,
which comprises reacting a compound of the formula:

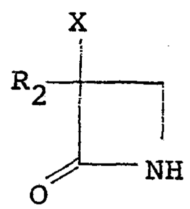

(II)

wherein $R_2$ stands for an amino group which is acylated or protected, and X is the same meaning as defined above, with a t-butyl or isopropylsilyl compound, followed by removing the protective group of $R_2$, if necessary, when $R_2$ is a protected amino group.

11. A method as claimed in Claim 10, wherein the t-butyl or isopropylsilyl compound is tert-butyldimethylchlorosilane or isopropyldimethylchlorosilane.

0053387

Application number

European Patent Office

# EUROPEAN SEARCH REPORT

EP 81 11 0039

| Category | Citation of document with indication, where appropriate, of relevant passages | Relevant to claim | CLASSIFICATION OF THE APPLICATION (Int. Cl. 3) |
|---|---|---|---|
| A | US - A - 4 224 336 (CHRISTENSEN et al.) * Columns 30,31 * | 7-11 | C 07 D 205/08<br>C 07 F 7/10<br>C 07 D 417/12 |
| | -- | | |
| A | EP - A - 0 001 628 (MERCK) * Page 54, example 13 * | 7-11 | |
| | -- | | |
| PA | EP - A - 0 021 678 (TAKEDA) * Claims 1,2,6 * | 1 | |
| | ---- | | |

TECHNICAL FIELDS SEARCHED (Int.Cl. 3)

C 07 D 205/00
C 07 F 7/00
C 07 D 417/00

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| The Hague | 18-02-1982 | CHOULY |

EPO Form 1503.1 06.78